# EUROPEAN PATENT SPECIFICATION

(11) **EP 0 887 412 B1**
(45) Date of publication and mention of the grant of the patent: **15.10.2003**
(21) Application number: 98201704.8
(22) Date of filing: 22.05.1998
(51) Int. Cl.: C12N 15/40, C12N 7/01, A61K 39/12, G01N 33/569

(54) **Recombinant birnavirus vaccine**
Rekombinanter Birnavirusimpfstoff
Vaccin recombinant du birnavirus

(30) Priority: 26.05.1997 EP 97201599
(43) Date of publication of application: 30.12.1998
(73) Proprietor: Akzo Nobel N.V., 6824 BM Arnhem (NL)
(72) Inventor: Mundt, E., 18461 Millienhagen (DE); Lütticken, H.D., 5831 CE Boxmeer (NL); van Loon, A.A.W.M., 5836 BB Sambeek (NL)
(74) Representative: Mestrom, Joannes Jozef Louis

(56) References cited:
- WO-A-95/26196
- E.MUNDT AND V.N.VAKHARIA: "Synthetic transcripts of double-stranded Birnavirus genome are infectious" PROCEEDINGS OF THE NATIONAL ACADEMY OF SCIENCES OF USA., vol. 93, October 1996, WASHINGTON US, pages 11131-11136, XP002044624
- E.MUNDT ET AL.: "Identification of a novel viral protein in infectious bursal disease virus-infected cells" JOURNAL OF GENERAL VIROLOGY, vol. 76, no. 2, February 1995, READING GB, pages 437-443, XP002044625
- E. MUNDT ET AL.: "VP5 of Infectious Bursal Disease Virus is not essential for virus replication in cell culture" JOURNAL OF VIROLOGY, vol. 71, no. 7, July 1997, pages 5647-5651, XP002044626
- MURPHY ET AL: 'PRODUCTION AND LEVEL OF GENETIC STABILITY OF AN INFLUENZA A VIRUS TEMPERATURE-SENSITIVE MUTANT CONTAINING TWO GENES WITH TW MUTATIONS' INFECTION AND IMMUNITY vol. 37, no. 1, 1982, pages 235 - 242
- Mundt E: An Abstract circulated at the Annual Meeting of the German Virological Society 1997 (Hamburg, 10-13 March 1997)
- Mundt E: A poster presented at the Annual Meeting of the German Virological Society 1997 (Hamburg, 10-13 March 1997)
- Mundt I: An Abstract in the Annual Report 1996 of the German Federal Research Institute for virus diseases in animal, published April 1997

### SEQUENCE LISTING

(1) GENERAL INFORMATION:
   (i) APPLICANT:
      (A) NAME: Azko Nobel N.V.
      (B) STREET: Velperweg 76
      (C) CITY: Arnhem
      (E) COUNTRY: The Netherlands
      (F) POSTAL CODE (ZIP): 6824 BM
      (G) TELEPHONE: 0412 666379
      (H) TELEFAX: 0412 650592
   (ii) TITLE OF INVENTION: Recombinant birnavirus vaccine
   (iii) NUMBER OF SEQUENCES: 8
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30 (EPO)
(2) INFORMATION FOR SEQ ID NO: 1:
   (i) SEQUENCE CHARACTERISTICS:
      (A) LENGTH: 2827 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION:112..2745
   (xi) SEQUENCE DESCRIPTION: SEQ ID NO: 1:
   GGATACGATG GGTCTGACCC TCTGGGAGTC ACGAATTAAC GTGGCTACTA GGGGCGATAC      60
   CCGCCGCTGG CCGCCACGTT AGTGGCTCCT CTTCTTGATG ATTCTGCCAC C ATG AGT 117
   Met Ser
   1
   GAC ATT TTC AAC AGT CCA CAG GCG CGA AGC ACG ATC TCA GCA GCG TTC 165
   Asp Ile Phe Asn Ser Pro Gln Ala Arg Ser Thr Ile Ser Ala Ala Phe
   5 10 15
   GGC ATA AAG CCT ACT GCT GGA CAA GAC GTG GAA GAA CTC TTG ATC CCT 213
   Gly Ile Lys Pro Thr Ala Gly Gln Asp Val Glu Glu Leu Leu Ile Pro
   20 25 30
   AAA GTT TGG GTG CCA CCT GAG GAT CCG CTT GCC AGC CCT AGT CGA CTG 261
   Lys Val Trp Val Pro Pro Glu Asp Pro Leu Ala Ser Pro Ser Arg Leu
   35 40 45 50
   GCA AAG TTC CTC AGA GAG AAC GGC TAC AAA GTT TTG CAG CCA CGG TCT 309
   Ala Lys Phe Leu Arg Glu Asn Gly Tyr Lys Val Leu Gln Pro Arg Ser
   55 60 65
   CTG CCC GAG AAT GAG GAG TAT GAG ACC GAC CAA ATA CTC CCA GAC TTA 357
   Leu Pro Glu Asn Glu Glu Tyr Glu Thr Asp Gln Ile Leu Pro Asp Leu
   70 75 80
   GCA TGG ATG CGA CAG ATA GAA GGG GCT GTT TTA AAA CCC ACT CTA TCT 405
   Ala Trp Met Arg Gln Ile Glu Gly Ala Val Leu Lys Pro Thr Leu Ser
   85 90 95
   CTC CCT ATT GGA GAT CAG GAG TAC TTC CCA AAG TAC TAC CCA ACA CAT 453
   Leu Pro Ile Gly Asp Gln Glu Tyr Phe Pro Lys Tyr Tyr Pro Thr His
   100 105 110
   CGC CCT AGC AAG GAG AAG CCC AAT GCG TAC CCG CCA GAC ATC GCA CTA 501
   Arg Pro Ser Lys Glu Lys Pro Asn Ala Tyr Pro Pro Asp Ile Ala Leu
   115 120 125 130
   CTC AAG CAG ATG ATT TAC CTG TTT CTC CAG GTT CCA GAG GCC AAC GAG 549
   Leu Lys Gln Met Ile Tyr Leu Phe Leu Gln Val Pro Glu Ala Asn Glu
   135 140 145
   GGC CTA AAG GAT GAA GTA ACC CTC TTG ACC CAA AAC ATA AGG GAC AAG 597
   Gly Leu Lys Asp Glu Val Thr Leu Leu Thr Gln Asn Ile Arg Asp Lys
   150 155 160
   GCC TAT GGA AGT GGG ACC TAC ATG GGA CAA GCA AAT CGA CTT GTG GCC 645
   Ala Tyr Gly Ser Gly Thr Tyr Met Gly Gln Ala Asn Arg Leu Val Ala
   165 170 175
   ATG AAG GAG GTC GCC ACT GGA AGA AAC CCA AAC AAG GAT CCT CTA AAG        693
   Met Lys Glu Val Ala Thr Gly Arg Asn Pro Asn Lys Asp Pro Leu Lys
       180                 185                 190
   CTT GGG TAC ACT TTT GAG AGC ATC GCG CAG CTA CTT GAC ATC ACA CTA        741
   Leu Gly Tyr Thr Phe Glu Ser Ile Ala Gln Leu Leu Asp Ile Thr Leu
   195                 200                 205                 210
   CCG GTA GGC CCA CCC GGT GAG GAT GAC AAG CCC TGG GTG CCA CTC ACA        789
   Pro Val Gly Pro Pro Gly Glu Asp Asp Lys Pro Trp Val Pro Leu Thr
                   215                 220                 225
   AGA GTG CCG TCA CGG ATG TTG GTG CTG ACG GGA GAC GTA GAT GGC GAC        837
   Arg Val Pro Ser Arg Met Leu Val Leu Thr Gly Asp Val Asp Gly Asp
               230                 235                 240
   TTT GAG GTT GAA GAT TAC CTT CCC AAA ATC AAC CTC AAG TCA TCA AGT        885
   Phe Glu Val Glu Asp Tyr Leu Pro Lys Ile Asn Leu Lys Ser Ser Ser
           245                 250                 255
   GGA CTA CCA TAT GTA GGT CGC ACC AAA GGA GAG ACA ATT GGC GAG ATG        933
   Gly Leu Pro Tyr Val Gly Arg Thr Lys Gly Glu Thr Ile Gly Glu Met
       260                 265                 270
   ATA GCT ATC TCA AAC CAG TTT CTC AGA GAG CTA TCA ACA CTG TTG AAG        981
   Ile Ala Ile Ser Asn Gln Phe Leu Arg Glu Leu Ser Thr Leu Leu Lys
   275                 280                 285                 290
   CAA GGT GCA GGG ACA AAG GGG TCA AAC AAG AAG AAG CTA CTC AGC ATG       1029
   Gln Gly Ala Gly Thr Lys Gly Ser Asn Lys Lys Lys Leu Leu Ser Met
                   295                 300                 305
   TTA AGT GAC TAT TGG TAC TTA TCA TGC GGG CTT TTG TTT CCA AAG GCT       1077
   Leu Ser Asp Tyr Trp Tyr Leu Ser Cys Gly Leu Leu Phe Pro Lys Ala
               310                 315                 320
   GAA AGG TAC GAC AAA AGT ACA TGG CTC ACC AAG ACC CGG AAC ATA TGG       1125
   Glu Arg Tyr Asp Lys Ser Thr Trp Leu Thr Lys Thr Arg Asn Ile Trp
           325                 330                 335
   TCA GCT CCA TCC CCA ACA CAC CTC ATG ATC TCT ATG ATC ACC TGG CCC       1173
   Ser Ala Pro Ser Pro Thr His Leu Met Ile Ser Met Ile Thr Trp Pro
       340                 345                 350
   GTG ATG TCC AAC AGC CCA AAT AAC GTG TTG AAC ATT GAA GGG TGT CCA       1221
   Val Met Ser Asn Ser Pro Asn Asn Val Leu Asn Ile Glu Gly Cys Pro
   355                 360                 365                 370
   TCA CTC TAC AAA TTC AAC CCG TTC AGA GGA GGG TTG AAC AGG ATC GTC      1269
   Ser Leu Tyr Lys Phe Asn Pro Phe Arg Gly Gly Leu Asn Arg Ile Val
                   375                 380                 385
   GAG TGG ATA TTG GCC CCG GAA GAA CCC AAG GCT CTT GTA TAT GCG GAC      1317
   Glu Trp Ile Leu Ala Pro Glu Glu Pro Lys Ala Leu Val Tyr Ala Asp
               390                 395                 400
   AAC ATA TAC ATT GTC CAC TCA AAC ACG TGG TAC TCA ATT GAC CTA GAG      1365
   Asn Ile Tyr Ile Val His Ser Asn Thr Trp Tyr Ser Ile Asp Leu Glu
           405                 410                 415
   AAG GGT GAG GCA AAC TGC ACT CGC CAA CAC ATG CAA GCC GCA ATG TAC      1413
   Lys Gly Glu Ala Asn Cys Thr Arg Gln His Met Gln Ala Ala Met Tyr
       420                 425                 430
   TAC ATA CTC ACC AGA GGG TGG TCA GAC AAC GGC GAC CCA ATG TTC AAT      1461
   Tyr Ile Leu Thr Arg Gly Trp Ser Asp Asn Gly Asp Pro Met Phe Asn
   435                 440                 445                 450
   CAA ACA TGG GCC ACC TTT GCC ATG AAC ATT GCC CCT GCT CTA GTG GTG      1509
   Gln Thr Trp Ala Thr Phe Ala Met Asn Ile Ala Pro Ala Leu Val Val
                   455                 460                 465
   GAC TCA TCG TGC CTG ATA ATG AAC CTG CAA ATT AAG ACC TAT GGT CAA      1557
   Asp Ser Ser Cys Leu Ile Met Asn Leu Gln Ile Lys Thr Tyr Gly Gln
               470                 475                 480
   GGC AGC GGG AAT GCA GCC ACG TTC ATC AAC AAC CAC CTC TTG AGC ACA      1605
   Gly Ser Gly Asn Ala Ala Thr Phe Ile Asn Asn His Leu Leu Ser Thr
           485                 490                 495
   CTA GTG CTT GAC CAG TGG AAC CTG ATG AGA CAG CCC AGA CCA GAC AGC      1653
   Leu Val Leu Asp Gln Trp Asn Leu Met Arg Gln Pro Arg Pro Asp Ser
       500                 505                 510
   GAG GAG TTC AAA TCA ATT GAG GAC AAG CTA GGT ATC AAC TTT AAG ATT      1701
   Glu Glu Phe Lys Ser Ile Glu Asp Lys Leu Gly Ile Asn Phe Lys Ile
   515                 520                 525                 530
   GAG AGG TCC ATT GAT GAT ATC AGG GGC AAG CTG AGA CAG CTT GTC CTC      1749
   Glu Arg Ser Ile Asp Asp Ile Arg Gly Lys Leu Arg Gln Leu Val Leu
                   535                 540                 545
   CTT GCA CAA CCA GGG TAC CTG AGT GGG GGG GTT GAA CCA GAA CAA TCC      1797
   Leu Ala Gln Pro Gly Tyr Leu Ser Gly Gly Val Glu Pro Glu Gln Ser
               550                 555                 560
   AGC CCA ACT GTT GAG CTT GAC CTA CTA GGG TGG TCA GCT ACA TAC AGC      1845
   Ser Pro Thr Val Glu Leu Asp Leu Leu Gly Trp Ser Ala Thr Tyr Ser
           565                 570                 575
   AAA GAT CTC GGG ATC TAT GTG CCG GTG CTT GAC AAG GAA CGC CTA TTT      1893
   Lys Asp Leu Gly Ile Tyr Val Pro Val Leu Asp Lys Glu Arg Leu Phe
       580                 585                 590
   TGT TCT GCT GCG TAT CCC AAG GGA GTA GAG AAC AAG AGT CTC AAG TCC      1941
   Cys Ser Ala Ala Tyr Pro Lys Gly Val Glu Asn Lys Ser Leu Lys Ser
   595                 600                 605                 610
   AAA GTC GGG ATC GAG CAG GCA TAC AAG GTA GTC AGG TAT GAG GCG TTG      1989
   Lys Val Gly Ile Glu Gln Ala Tyr Lys Val Val Arg Tyr Glu Ala Leu
                   615                 620                 625
   AGG TTG GTA GGT GGT TGG AAC TAC CCA CTC CTG AAC AAA GCC TGC AAG      2037
   Arg Leu Val Gly Gly Trp Asn Tyr Pro Leu Leu Asn Lys Ala Cys Lys
               630                 635                 640
   AAT AAC GCA GGC GCC GCT CGG CGG CAT CTG GAG GCC AAG GGG TTC CCA      2085
   Asn Asn Ala Gly Ala Ala Arg Arg His Leu Glu Ala Lys Gly Phe Pro
           645                 650                 655
   CTC GAC GAG TTC CTA GCC GAG TGG TCT GAG CTG TCA GAG TTC GGT GAG      2133
   Leu Asp Glu Phe Leu Ala Glu Trp Ser Glu Leu Ser Glu Phe Gly Glu
       660                 665                 670
   GCC TTC GAA GGC TTC AAT ATC AAG CTG ACC GTA ACA TCT GAG AGC CTA      2181
   Ala Phe Glu Gly Phe Asn Ile Lys Leu Thr Val Thr Ser Glu Ser Leu
   675                 680                 685                 690
   GCC GAA CTG AAC AAG CCA GTA CCC CCC AAG CCC CCA AAT GTC AAC AGA      2229
   Ala Glu Leu Asn Lys Pro Val Pro Pro Lys Pro Pro Asn Val Asn Arg
                   695                 700                 705
   CCA GTC AAC ACT GGG GGA CTC AAG GCA GTC AGC AAC GCC CTC AAG ACC      2277
   Pro Val Asn Thr Gly Gly Leu Lys Ala Val Ser Asn Ala Leu Lys Thr
               710                 715                 720
   GGT CGG TAC AGG AAC GAA GCC GGA CTG AGT GGT CTC GTC CTT CTA GCC      2325
   Gly Arg Tyr Arg Asn Glu Ala Gly Leu Ser Gly Leu Val Leu Leu Ala
           725                 730                 735
   ACA GCA AGA AGC CGT CTG CAA GAT GCA GTT AAG GCC AAG GCA GAA GCC      2373
   Thr Ala Arg Ser Arg Leu Gln Asp Ala Val Lys Ala Lys Ala Glu Ala
       740                 745                 750
   GAG AAA CTC CAC AAG TCC AAG CCA GAC GAC CCC GAT GCA GAC TGG TTC      2421
   Glu Lys Leu His Lys Ser Lys Pro Asp Asp Pro Asp Ala Asp Trp Phe
   755                 760                 765                 770
   GAA AGA TCA GAA ACT CTG TCA GAC CTT CTG GAG AAA GCC GAC ATC GCC      2469
   Glu Arg Ser Glu Thr Leu Ser Asp Leu Leu Glu Lys Ala Asp Ile Ala
                   775                 780                 785
   AGC AAG GTC GCC CAC TCA GCA CTC GTG GAA ACA AGC GAC GCC CTT GAA      2517
   Ser Lys Val Ala His Ser Ala Leu Val Glu Thr Ser Asp Ala Leu Glu
               790                 795                 800
   GCA GTT CAG TCG ACT TCC GTG TAC ACC CCC AAG TAC CCA GAA GTC AAG      2565
   Ala Val Gln Ser Thr Ser Val Tyr Thr Pro Lys Tyr Pro Glu Val Lys
           805                 810                 815
   AAC CCA CAG ACC GCC TCC AAC CCC GTT GTT GGG CTC CAC CTG CCC GCC      2613
   Asn Pro Gln Thr Ala Ser Asn Pro Val Val Gly Leu His Leu Pro Ala
       820                 825                 830
   AAG AGA GCC ACC GGT GTC CAG GCC GCT CTT CTC GGA GCA GGA ACG AGC      2661
   Lys Arg Ala Thr Gly Val Gln Ala Ala Leu Leu Gly Ala Gly Thr Ser
   835                 840                 845                 850
   AGA CCA ATG GGG ATG GAG GCC CCA ACA CGG TCC AAG AAC GCC GTG AAA      2709
   Arg Pro Met Gly Met Glu Ala Pro Thr Arg Ser Lys Asn Ala Val Lys
                   855                 860                 865
   ATG GCC AAA CGG CGG CAA CGC CAA AAG GAG AGC CGC TAACAGCCAT           2755
   Met Ala Lys Arg Arg Gln Arg Gln Lys Glu Ser Arg
               870                 875
   GATGGGAACC ACTCAAGAAG AGGACACTAA TCCCAGACCC CGTATCCCCG GCCTTCGCCT    2815
   GCGGGGGCCC CC                                                        2827
(2) INFORMATION FOR SEQ ID NO: 2:
   (i) SEQUENCE CHARACTERISTICS:
      (A) LENGTH: 878 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Ser Asp Ile Phe Asn Ser Pro Gln Ala Arg Ser Thr Ile Ser Ala
   1 5 10 15
   Ala Phe Gly Ile Lys Pro Thr Ala Gly Gln Asp Val Glu Glu Leu Leu
   20 25 30
   Ile Pro Lys Val Trp Val Pro Pro Glu Asp Pro Leu Ala Ser Pro Ser
   35 40 45
   Arg Leu Ala Lys Phe Leu Arg Glu Asn Gly Tyr Lys Val Leu Gln Pro
   50 55 60
   Arg Ser Leu Pro Glu Asn Glu Glu Tyr Glu Thr Asp Gln Ile Leu Pro
   65 70 75 80
   Asp Leu Ala Trp Met Arg Gln Ile Glu Gly Ala Val Leu Lys Pro Thr
   85 90 95
   Leu Ser Leu Pro Ile Gly Asp Gln Glu Tyr Phe Pro Lys Tyr Tyr Pro
   100 105 110
   Thr His Arg Pro Ser Lys Glu Lys Pro Asn Ala Tyr Pro Pro Asp Ile
   115 120 125
   Ala Leu Leu Lys Gln Met Ile Tyr Leu Phe Leu Gln Val Pro Glu Ala
   130 135 140
   Asn Glu Gly Leu Lys Asp Glu Val Thr Leu Leu Thr Gln Asn Ile Arg
   145 150 155 160
   Asp Lys Ala Tyr Gly Ser Gly Thr Tyr Met Gly Gln Ala Asn Arg Leu
   165 170 175
   Val Ala Met Lys Glu Val Ala Thr Gly Arg Asn Pro Asn Lys Asp Pro
   180 185 190
   Leu Lys Leu Gly Tyr Thr Phe Glu Ser Ile Ala Gln Leu Leu Asp Ile
   195 200 205
   Thr Leu Pro Val Gly Pro Pro Gly Glu Asp Asp Lys Pro Trp Val Pro
   210 215 220
   Leu Thr Arg Val Pro Ser Arg Met Leu Val Leu Thr Gly Asp Val Asp
   225 230 235 240
   Gly Asp Phe Glu Val Glu Asp Tyr Leu Pro Lys Ile Asn Leu Lys Ser
   245 250 255
   Ser Ser Gly Leu Pro Tyr Val Gly Arg Thr Lys Gly Glu Thr Ile Gly
               260                 265                 270
   Glu Met Ile Ala Ile Ser Asn Gln Phe Leu Arg Glu Leu Ser Thr Leu
           275                 280                 285
   Leu Lys Gln Gly Ala Gly Thr Lys Gly Ser Asn Lys Lys Lys Leu Leu
       290                 295                 300
   Ser Met Leu Ser Asp Tyr Trp Tyr Leu Ser Cys Gly Leu Leu Phe Pro
   305                 310                 315                 320
   Lys Ala Glu Arg Tyr Asp Lys Ser Thr Trp Leu Thr Lys Thr Arg Asn
                   325                 330                 335
   Ile Trp Ser Ala Pro Ser Pro Thr His Leu Met Ile Ser Met Ile Thr
               340                 345                 350
   Trp Pro Val Met Ser Asn Ser Pro Asn Asn Val Leu Asn Ile Glu Gly
           355                 360                 365
   Cys Pro Ser Leu Tyr Lys Phe Asn Pro Phe Arg Gly Gly Leu Asn Arg
       370                 375                 380
   Ile Val Glu Trp Ile Leu Ala Pro Glu Glu Pro Lys Ala Leu Val Tyr
   385                 390                 395                 400
   Ala Asp Asn Ile Tyr Ile Val His Ser Asn Thr Trp Tyr Ser Ile Asp
                   405                 410                 415
   Leu Glu Lys Gly Glu Ala Asn Cys Thr Arg Gln His Met Gln Ala Ala
               420                 425                 430
   Met Tyr Tyr Ile Leu Thr Arg Gly Trp Ser Asp Asn Gly Asp Pro Met
           435                 440                 445
   Phe Asn Gln Thr Trp Ala Thr Phe Ala Met Asn Ile Ala Pro Ala Leu
       450                 455                 460
   Val Val Asp Ser Ser Cys Leu Ile Met Asn Leu Gln Ile Lys Thr Tyr
   465                 470                 475                 480
   Gly Gln Gly Ser Gly Asn Ala Ala Thr Phe Ile Asn Asn His Leu Leu
                   485                 490                 495
   Ser Thr Leu Val Leu Asp Gln Trp Asn Leu Met Arg Gln Pro Arg Pro
               500                 505                 510
   Asp Ser Glu Glu Phe Lys Ser Ile Glu Asp Lys Leu Gly Ile Asn Phe
   515 520 525
   Lys Ile Glu Arg Ser Ile Asp Asp Ile Arg Gly Lys Leu Arg Gln Leu
   530 535 540
   Val Leu Leu Ala Gln Pro Gly Tyr Leu Ser Gly Gly Val Glu Pro Glu
   545 550 555 560
   Gln Ser Ser Pro Thr Val Glu Leu Asp Leu Leu Gly Trp Ser Ala Thr
   565 570 575
   Tyr Ser Lys Asp Leu Gly Ile Tyr Val Pro Val Leu Asp Lys Glu Arg
   580 585 590
   Leu Phe Cys Ser Ala Ala Tyr Pro Lys Gly Val Glu Asn Lys Ser Leu
   595 600 605
   Lys Ser Lys Val Gly Ile Glu Gln Ala Tyr Lys Val Val Arg Tyr Glu
   610 615 620
   Ala Leu Arg Leu Val Gly Gly Trp Asn Tyr Pro Leu Leu Asn Lys Ala
   625 630 635 640
   Cys Lys Asn Asn Ala Gly Ala Ala Arg Arg His Leu Glu Ala Lys Gly
   645 650 655
   Phe Pro Leu Asp Glu Phe Leu Ala Glu Trp Ser Glu Leu Ser Glu Phe
   660 665 670
   Gly Glu Ala Phe Glu Gly Phe Asn Ile Lys Leu Thr Val Thr Ser Glu
   675 680 685
   Ser Leu Ala Glu Leu Asn Lys Pro Val Pro Pro Lys Pro Pro Asn Val
   690 695 700
   Asn Arg Pro Val Asn Thr Gly Gly Leu Lys Ala Val Ser Asn Ala Leu
   705 710 715 720
   Lys Thr Gly Arg Tyr Arg Asn Glu Ala Gly Leu Ser Gly Leu Val Leu
   725 730 735
   Leu Ala Thr Ala Arg Ser Arg Leu Gln Asp Ala Val Lys Ala Lys Ala
   740 745 750
   Glu Ala Glu Lys Leu His Lys Ser Lys Pro Asp Asp Pro Asp Ala Asp
   755 760 765
   Trp Phe Glu Arg Ser Glu Thr Leu Ser Asp Leu Leu Glu Lys Ala Asp
       770                 775                 780
   Ile Ala Ser Lys Val Ala His Ser Ala Leu Val Glu Thr Ser Asp Ala
   785                 790                 795                 800
   Leu Glu Ala Val Gln Ser Thr Ser Val Tyr Thr Pro Lys Tyr Pro Glu
                   805                 810                 815
   Val Lys Asn Pro Gln Thr Ala Ser Asn Pro Val Val Gly Leu His Leu
               820                 825                 830
   Pro Ala Lys Arg Ala Thr Gly Val Gln Ala Ala Leu Leu Gly Ala Gly
           835                 840                 845
   Thr Ser Arg Pro Met Gly Met Glu Ala Pro Thr Arg Ser Lys Asn Ala
       850                 855                 860
   Val Lys Met Ala Lys Arg Arg Gln Arg Gln Lys Glu Ser Arg
   865                 870                 875
(2) INFORMATION FOR SEQ ID NO: 3:
   (i) SEQUENCE CHARACTERISTICS:
      (A) LENGTH: 3261 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION:97..531
   (xi) SEQUENCE DESCRIPTION: SEQ ID NO: 3:
   GGATACGATC GGTCTGACCC CGGGGGAGTC ACCCGGGGAC AGGCCGTCAA GGCCTTGTTC      60
   CAGGATGGGA CTCCTCCTTC TACAACGCTA TCATTG ATG GTT AGT AGA GAT CAG       114
                                           Met Val Ser Arg Asp Gln
                                            1               5
   ACA AAC GAT CGC AGC GAT GAC AAA CCT GCA AGA TCA AAC CCA ACA GAT      162
   Thr Asn Asp Arg Ser Asp Asp Lys Pro Ala Arg Ser Asn Pro Thr Asp
               10                  15                  20
   TGT TCC GTT CAT ACG GAG CCT TCT GAT GCC AAC AAC CGG ACC GGC GTC      210
   Cys Ser Val His Thr Glu Pro Ser Asp Ala Asn Asn Arg Thr Gly Val
           25                  30                  35
   CAT TCC GGA CGA CAC CCT GGA GAA GCA CAC TCT CAG GTC AGA GAC CTC      258
   His Ser Gly Arg His Pro Gly Glu Ala His Ser Gln Val Arg Asp Leu
        40                  45                  50
   GAC CTA CAA TTT GAC TGT GGG GGA CAC AGG GTC AGG GCT AAT TGT CTT      306
   Asp Leu Gln Phe Asp Cys Gly Gly His Arg Val Arg Ala Asn Cys Leu
    55                  60                  65                  70
   TTT CCC TGG ATT CCC TGG CTC AAT TGT GGG TGC TCA CTA CAC ACT GCA      354
   Phe Pro Trp Ile Pro Trp Leu Asn Cys Gly Cys Ser Leu His Thr Ala
                   75                  80                  85
   GGG CAA TGG GAA CTA CAA GTT CGA TCA GAT GCT CCT GAC TGC CCA GAA      402
   Gly Gln Trp Glu Leu Gln Val Arg Ser Asp Ala Pro Asp Cys Pro Glu
               90                  95                 100
   CCT ACC GGC CAG TTA CAA CTA CTG CAG GCT AGT GAG TCG GAG TCT CAC      450
   Pro Thr Gly Gln Leu Gln Leu Leu Gln Ala Ser Glu Ser Glu Ser His
           105                 110                 115
   AGT GAG GTC AAG CAC ACT TCC TGG TGG CGT TTA TGC ACT AAA CGG CAC      498
   Ser Glu Val Lys His Thr Ser Trp Trp Arg Leu Cys Thr Lys Arg His
       120                 125                 130
   CAT AAA CGC CGT GAC CTT CCA AGG AAG CCT GAG TGAACTGACA GATGTTAGCT    551
   His Lys Arg Arg Asp Leu Pro Arg Lys Pro Glu
   135                 140                 145
   ACAATGGGTT GATGTCTGCA ACAGCCAACA TCAACGACAA AATTGGGAAC GTCCTAGTAG    611
   GGGAAGGGGT CACCGTCCTC AGCTTACCCA CATCATATGA TCTTGGGTAT GTGAGGCTTG    671
   GTGACCCCAT TCCCGCAATA GGGCTTGACC CAAAAATGGT AGCCACATGT GACAGCAGTG    731
   ACAGGCCCAG AGTCTACACC ATAACTGCAG CCGATGATTA CCAATTCTCA TCACAGTACC    791
   AACCAGGTGG GGTAACAATC ACACTGTTCT CAGCCAACAT TGATGCCATC ACAAGCCTCA    851
   GCGTTGGGGG AGAGCTCGTG TTTCAAACAA GCGTCCACGG CCTTGTACTG GGCGCCACCA    911
   TCTACCTCAT AGGCTTTGAT GGGACAACGG TAATCACCAG GGCTGTGGCC GCAAACAATG 971
   GGCTGACGAC CGGCACCGAC AACCTTATGC CATTCAATCT TGTGATTCCA ACAAACGAGA 1031
   TAACCCAGCC AATCACATCC ATCAAACTGG AGATAGTGAC CTCCAAAAGT GGTGGTCAGG 1091
   CAGGGGATCA GATGTCATGG TCGGCAAGAG GGAGCCTAGC AGTGACGATC CATGGTGGCA 1151
   ACTATCCAGG GGCCCTCCGT CCCGTCACGC TAGTGGCCTA CGAAAGAGTG GCAACAGGAT 1211
   CCGTCGTTAC GGTCGCTGGG GTGAGCAACT TCGAGCTGAT CCCAAATCCT GAACTAGCAA 1271
   AGAACCTGGT TACAGAATAC GGCCGATTTG ACCCAGGAGC CATGAACTAC ACAAAATTGA 1331
   TACTGAGTGA GAGGGACCGT CTTGGCATCA AGACCGTCTG GCCAACAAGG GAGTACACTG 1391
   ACTTTCGTGA ATACTTCATG GAGGTGGCCG ACCTCAACTC TCCCCTGAAG ATTGCAGGAG 1451
   CATTCGGCTT CAAAGACATA ATCCGGGCCA TAAGGAGGAT AGCTGTGCCG GTGGTCTCCA 1511
   CATTGTTCCC ACCTGCCGCT CCCCTAGCCC ATGCAATTGG GGAAGGTGTA GACTACCTGC 1571
   TGGGCGATGA GGCACAGGCT GCTTCAGGAA CTGCTCGAGC CGCGTCAGGA AAAGCAAGAG 1631
   CTGCCTCAGG CCGCATAAGG CAGCTGACTC TCGCCGCCGA CAAGGGGTAC GAGGTAGTCG 1691
   CGAATCTATT CCAGGTGCCC CAGAATCCCG TAGTCGACGG GATTCTTGCT TCACCTGGGG 1751
   TACTCCGCGG TGCACACAAC CTCGACTGCG TGTTAAGAGA GGGTGCCACG CTATTCCCTG 1811
   TGGTTATTAC GACAGTGGAA GACGCCATGA CACCCAAAGC ATTGAACAGC AAAATGTTTG 1871
   CTGTCATTGA AGGCGTGCGA GAAGACCTCC AACCTCCATC TCAAAGAGGA TCCTTCATAC 1931
   GAACTCTCTC TGGACACAGA GTCTATGGAT ATGCTCCAGA TGGGGTACTT CCACTGGAGA 1991
   CTGGGAGAGA CTACACCGTT GTCCCAATAG ATGATGTCTG GGACGACAGC ATTATGCTGT 2051
   CCAAAGATCC CATACCTCCT ATTGTGGGAA ACAGTGGAAA TCTAGCCATA GCTTACATGG 2111
   ATGTGTTTCG ACCCAAAGTC CCAATCCATG TGGCTATGAC GGGAGCCCTC AATGCTTGTG 2171
   GCGAGATTGA GAAAGTAAGC TTTAGAAGCA CCAAGCTCGC CACTGCACAC CGACTTGGCC 2231
   TTAGGTTGGC TGGTCCCGGA GCATTCGATG TAAACACCGG GCCCAACTGG GCAACGTTCA 2291
   TCAAACGTTT CCCTCACAAT CCACGCGACT GGGACAGGCT CCCCTACCTC AACCTACCAT 2351
   ACCTTCCACC CAATGCAGGA CGCCAGTACC ACCTTGCCAT GGCTGCATCA GAGTTCAAAG    2411
   AGACCCCCGA ACTCGAGAGT GCCGTCAGAG CAATGGAAGC AGCAGCCAAC GTGGACCCAC    2471
   TATTCCAATC TGCACTCAGT GTGTTCATGT GGCTGGAAGA GAATGGGATT GTGACTGACA    2531
   TGGCCAACTT CGCACTCAGC GACCCGAACG CCCATCGGAT GCGAAATTTT CTTGCAAACG    2591
   CACCACAAGC AGGCAGCAAG TCGCAAAGGG CCAAGTACGG GACAGCAGGC TACGGAGTGG    2651
   AGGCTCGGGG CCCCACACCA GAGGAAGCAC AGAGGGAAAA AGACACACGG ATCTCAAAGA    2711
   AGATGGAGAC CATGGGCATC TACTTTGCAA CACCAGAATG GGTAGCACTC AATGGGCACC    2771
   GAGGGCCAAG CCCCGGCCAG CTAAAGTACT GGCAGAACAC ACGAGAAATA CCGGACCCAA    2831
   ACGAGGACTA TCTAGACTAC GTGCATGCAG AGAAGAGCCG GTTGGCATCA GAAGAACAAA    2891
   TCCTAAGGGC AGCTACGTCG ATCTACGGGG CTCCAGGACA GGCAGAGCCA CCCCAAGCTT    2951
   TCATAGACGA AGTTGCCAAA GTCTATGAAA TCAACCATGG ACGTGGCCCA AACCAAGAAC    3011
   AGATGAAAGA TCTGCTCTTG ACTGCGATGG AGATGAAGCA TCGCAATCCC AGGCGGGCTC    3071
   TACCAAAGCC CAAGCCAAAA CCCAATGCTC CAACACAGAG ACCCCCTGGT CGGCTGGGCC    3131
   GCTGGATCAG GACCGTCTCT GATGAGGACC TTGAGTGAGG CTCCTGGGAG TCTCCCGACA    3191
   CCACCCGCGC AGGTGTGGAC ACCAATTCGG CCTTACAACA TCCCAAATTG GATCCGTTCG    3251
   CGGGTCCCCT                                                           3261
(2) INFORMATION FOR SEQ ID NO: 4:
   (i) SEQUENCE CHARACTERISTICS:
      (A) LENGTH: 145 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Val Ser Arg Asp Gln Thr Asn Asp Arg Ser Asp Asp Lys Pro Ala
    1               5                  10                  15
   Arg Ser Asn Pro Thr Asp Cys Ser Val His Thr Glu Pro Ser Asp Ala
               20                  25                  30
   Asn Asn Arg Thr Gly Val His Ser Gly Arg His Pro Gly Glu Ala His
           35                  40                  45
   Ser Gln Val Arg Asp Leu Asp Leu Gln Phe Asp Cys Gly Gly His Arg
       50                  55                  60
   Val Arg Ala Asn Cys Leu Phe Pro Trp Ile Pro Trp Leu Asn Cys Gly
   65                  70                  75                  80
   Cys Ser Leu His Thr Ala Gly Gln Trp Glu Leu Gln Val Arg Ser Asp
                   85                  90                  95
   Ala Pro Asp Cys Pro Glu Pro Thr Gly Gln Leu Gln Leu Leu Gln Ala
               100                 105                 110
   Ser Glu Ser Glu Ser His Ser Glu Val Lys His Thr Ser Trp Trp Arg
           115                 120                 125
   Leu Cys Thr Lys Arg His His Lys Arg Arg Asp Leu Pro Arg Lys Pro
       130                 135                 140
   Glu
   145
(2) INFORMATION FOR SEQ ID NO: 5:
   (i) SEQUENCE CHARACTERISTICS:
      (A) LENGTH: 3261 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION:131..3166
   (xi) SEQUENCE DESCRIPTION: SEQ ID NO: 5:
   GGATACGATC GGTCTGACCC CGGGGGAGTC ACCCGGGGAC AGGCCGTCAA GGCCTTGTTC      60
   CAGGATGGGA CTCCTCCTTC TACAACGCTA TCATTGATGG TTAGTAGAGA TCAGACAAAC    120
   GATCGCAGCG ATG ACA AAC CTG CAA GAT CAA ACC CAA CAG ATT GTT CCG       169
              Met Thr Asn Leu Gln Asp Gln Thr Gln Gln Ile Val Pro
                1               5                  10
   TTC ATA CGG AGC CTT CTG ATG CCA ACA ACC GGA CCG GCG TCC ATT CCG      217
   Phe Ile Arg Ser Leu Leu Met Pro Thr Thr Gly Pro Ala Ser Ile Pro
        15                  20                  25
   GAC GAC ACC CTG GAG AAG CAC ACT CTC AGG TCA GAG ACC TCG ACC TAC      265
   Asp Asp Thr Leu Glu Lys His Thr Leu Arg Ser Glu Thr Ser Thr Tyr
    30                  35                  40                  45
   AAT TTG ACT GTG GGG GAC ACA GGG TCA GGG CTA ATT GTC TTT TTC CCT      313
   Asn Leu Thr Val Gly Asp Thr Gly Ser Gly Leu Ile Val Phe Phe Pro
                    50                  55                  60
   GGA TTC CCT GGC TCA ATT GTG GGT GCT CAC TAC ACA CTG CAG GGC AAT      361
   Gly Phe Pro Gly Ser Ile Val Gly Ala His Tyr Thr Leu Gln Gly Asn
                65                  70                  75
   GGG AAC TAC AAG TTC GAT CAG ATG CTC CTG ACT GCC CAG AAC CTA CCG      409
   Gly Asn Tyr Lys Phe Asp Gln Met Leu Leu Thr Ala Gln Asn Leu Pro
            80                  85                  90
   GCC AGT TAC AAC TAC TGC AGG CTA GTG AGT CGG AGT CTC ACA GTG AGG      457
   Ala Ser Tyr Asn Tyr Cys Arg Leu Val Ser Arg Ser Leu Thr Val Arg
        95                 100                 105
   TCA AGC ACA CTT CCT GGT GGC GTT TAT GCA CTA AAC GGC ACC ATA AAC      505
   Ser Ser Thr Leu Pro Gly Gly Val Tyr Ala Leu Asn Gly Thr Ile Asn
   110                 115                 120                 125
   GCC GTG ACC TTC CAA GGA AGC CTG AGT GAA CTG ACA GAT GTT AGC TAC      553
   Ala Val Thr Phe Gln Gly Ser Leu Ser Glu Leu Thr Asp Val Ser Tyr
                   130                 135                 140
   AAT GGG TTG ATG TCT GCA ACA GCC AAC ATC AAC GAC AAA ATT GGG AAC      601
   Asn Gly Leu Met Ser Ala Thr Ala Asn Ile Asn Asp Lys Ile Gly Asn
               145                 150                 155
   GTC CTA GTA GGG GAA GGG GTC ACC GTC CTC AGC TTA CCC ACA TCA TAT      649
   Val Leu Val Gly Glu Gly Val Thr Val Leu Ser Leu Pro Thr Ser Tyr
           160                 165                 170
   GAT CTT GGG TAT GTG AGG CTT GGT GAC CCC ATT CCC GCA ATA GGG CTT      697
   Asp Leu Gly Tyr Val Arg Leu Gly Asp Pro Ile Pro Ala Ile Gly Leu
       175                 180                 185
   GAC CCA AAA ATG GTA GCC ACA TGT GAC AGC AGT GAC AGG CCC AGA GTC      745
   Asp Pro Lys Met Val Ala Thr Cys Asp Ser Ser Asp Arg Pro Arg Val
   190                 195                 200                 205
   TAC ACC ATA ACT GCA GCC GAT GAT TAC CAA TTC TCA TCA CAG TAC CAA      793
   Tyr Thr Ile Thr Ala Ala Asp Asp Tyr Gln Phe Ser Ser Gln Tyr Gln
                   210                 215                 220
   CCA GGT GGG GTA ACA ATC ACA CTG TTC TCA GCC AAC ATT GAT GCC ATC      841
   Pro Gly Gly Val Thr Ile Thr Leu Phe Ser Ala Asn Ile Asp Ala Ile
               225                 230                 235
   ACA AGC CTC AGC GTT GGG GGA GAG CTC GTG TTT CAA ACA AGC GTC CAC      889
   Thr Ser Leu Ser Val Gly Gly Glu Leu Val Phe Gln Thr Ser Val His
           240                 245                 250
   GGC CTT GTA CTG GGC GCC ACC ATC TAC CTC ATA GGC TTT GAT GGG ACA      937
   Gly Leu Val Leu Gly Ala Thr Ile Tyr Leu Ile Gly Phe Asp Gly Thr
       255                 260                 265
   ACG GTA ATC ACC AGG GCT GTG GCC GCA AAC AAT GGG CTG ACG ACC GGC      985
   Thr Val Ile Thr Arg Ala Val Ala Ala Asn Asn Gly Leu Thr Thr Gly
   270                 275                 280                 285
   ACC GAC AAC CTT ATG CCA TTC AAT CTT GTG ATT CCA ACA AAC GAG ATA     1033
   Thr Asp Asn Leu Met Pro Phe Asn Leu Val Ile Pro Thr Asn Glu Ile
                   290                 295                 300
   ACC CAG CCA ATC ACA TCC ATC AAA CTG GAG ATA GTG ACC TCC AAA AGT     1081
   Thr Gln Pro Ile Thr Ser Ile Lys Leu Glu Ile Val Thr Ser Lys Ser
               305                 310                 315
   GGT GGT CAG GCA GGG GAT CAG ATG TCA TGG TCG GCA AGA GGG AGC CTA     1129
   Gly Gly Gln Ala Gly Asp Gln Met Ser Trp Ser Ala Arg Gly Ser Leu
           320                 325                 330
   GCA GTG ACG ATC CAT GGT GGC AAC TAT CCA GGG GCC CTC CGT CCC GTC     1177
   Ala Val Thr Ile His Gly Gly Asn Tyr Pro Gly Ala Leu Arg Pro Val
       335                 340                 345
   ACG CTA GTG GCC TAC GAA AGA GTG GCA ACA GGA TCC GTC GTT ACG GTC     1225
   Thr Leu Val Ala Tyr Glu Arg Val Ala Thr Gly Ser Val Val Thr Val
   350                 355                 360                 365
   GCT GGG GTG AGC AAC TTC GAG CTG ATC CCA AAT CCT GAA CTA GCA AAG     1273
   Ala Gly Val Ser Asn Phe Glu Leu Ile Pro Asn Pro Glu Leu Ala Lys
                   370                 375                 380
   AAC CTG GTT ACA GAA TAC GGC CGA TTT GAC CCA GGA GCC ATG AAC TAC      1321
   Asn Leu Val Thr Glu Tyr Gly Arg Phe Asp Pro Gly Ala Met Asn Tyr
               385                 390                 395
   ACA AAA TTG ATA CTG AGT GAG AGG GAC CGT CTT GGC ATC AAG ACC GTC      1369
   Thr Lys Leu Ile Leu Ser Glu Arg Asp Arg Leu Gly Ile Lys Thr Val
           400                 405                 410
   TGG CCA ACA AGG GAG TAC ACT GAC TTT CGT GAA TAC TTC ATG GAG GTG      1417
   Trp Pro Thr Arg Glu Tyr Thr Asp Phe Arg Glu Tyr Phe Met Glu Val
       415                 420                 425
   GCC GAC CTC AAC TCT CCC CTG AAG ATT GCA GGA GCA TTC GGC TTC AAA      1465
   Ala Asp Leu Asn Ser Pro Leu Lys Ile Ala Gly Ala Phe Gly Phe Lys
   430                 435                 440                 445
   GAC ATA ATC CGG GCC ATA AGG AGG ATA GCT GTG CCG GTG GTC TCC ACA      1513
   Asp Ile Ile Arg Ala Ile Arg Arg Ile Ala Val Pro Val Val Ser Thr
                   450                 455                 460
   TTG TTC CCA CCT GCC GCT CCC CTA GCC CAT GCA ATT GGG GAA GGT GTA      1561
   Leu Phe Pro Pro Ala Ala Pro Leu Ala His Ala Ile Gly Glu Gly Val
               465                 470                 475
   GAC TAC CTG CTG GGC GAT GAG GCA CAG GCT GCT TCA GGA ACT GCT CGA      1609
   Asp Tyr Leu Leu Gly Asp Glu Ala Gln Ala Ala Ser Gly Thr Ala Arg
           480                 485                 490
   GCC GCG TCA GGA AAA GCA AGA GCT GCC TCA GGC CGC ATA AGG CAG CTG      1657
   Ala Ala Ser Gly Lys Ala Arg Ala Ala Ser Gly Arg Ile Arg Gln Leu
       495                 500                 505
   ACT CTC GCC GCC GAC AAG GGG TAC GAG GTA GTC GCG AAT CTA TTC CAG      1705
   Thr Leu Ala Ala Asp Lys Gly Tyr Glu Val Val Ala Asn Leu Phe Gln
   510                 515                 520                 525
   GTG CCC CAG AAT CCC GTA GTC GAC GGG ATT CTT GCT TCA CCT GGG GTA      1753
   Val Pro Gln Asn Pro Val Val Asp Gly Ile Leu Ala Ser Pro Gly Val
                   530                 535                 540
   CTC CGC GGT GCA CAC AAC CTC GAC TGC GTG TTA AGA GAG GGT GCC ACG      1801
   Leu Arg Gly Ala His Asn Leu Asp Cys Val Leu Arg Glu Gly Ala Thr
               545                 550                 555
   CTA TTC CCT GTG GTT ATT ACG ACA GTG GAA GAC GCC ATG ACA CCC AAA      1849
   Leu Phe Pro Val Val Ile Thr Thr Val Glu Asp Ala Met Thr Pro Lys
           560                 565                 570
   GCA TTG AAC AGC AAA ATG TTT GCT GTC ATT GAA GGC GTG CGA GAA GAC      1897
   Ala Leu Asn Ser Lys Met Phe Ala Val Ile Glu Gly Val Arg Glu Asp
       575                 580                 585
   CTC CAA CCT CCA TCT CAA AGA GGA TCC TTC ATA CGA ACT CTC TCT GGA      1945
   Leu Gln Pro Pro Ser Gln Arg Gly Ser Phe Ile Arg Thr Leu Ser Gly
   590                 595                 600                 605
   CAC AGA GTC TAT GGA TAT GCT CCA GAT GGG GTA CTT CCA CTG GAG ACT      1993
   His Arg Val Tyr Gly Tyr Ala Pro Asp Gly Val Leu Pro Leu Glu Thr
                   610                 615                 620
   GGG AGA GAC TAC ACC GTT GTC CCA ATA GAT GAT GTC TGG GAC GAC AGC      2041
   Gly Arg Asp Tyr Thr Val Val Pro Ile Asp Asp Val Trp Asp Asp Ser
               625                 630                 635
   ATT ATG CTG TCC AAA GAT CCC ATA CCT CCT ATT GTG GGA AAC AGT GGA      2089
   Ile Met Leu Ser Lys Asp Pro Ile Pro Pro Ile Val Gly Asn Ser Gly
           640                 645                 650
   AAT CTA GCC ATA GCT TAC ATG GAT GTG TTT CGA CCC AAA GTC CCA ATC      2137
   Asn Leu Ala Ile Ala Tyr Met Asp Val Phe Arg Pro Lys Val Pro Ile
       655                 660                 665
   CAT GTG GCT ATG ACG GGA GCC CTC AAT GCT TGT GGC GAG ATT GAG AAA      2185
   His Val Ala Met Thr Gly Ala Leu Asn Ala Cys Gly Glu Ile Glu Lys
   670                 675                 680                 685
   GTA AGC TTT AGA AGC ACC AAG CTC GCC ACT GCA CAC CGA CTT GGC CTT      2233
   Val Ser Phe Arg Ser Thr Lys Leu Ala Thr Ala His Arg Leu Gly Leu
                   690                 695                 700
   AGG TTG GCT GGT CCC GGA GCA TTC GAT GTA AAC ACC GGG CCC AAC TGG      2281
   Arg Leu Ala Gly Pro Gly Ala Phe Asp Val Asn Thr Gly Pro Asn Trp
               705                 710                 715
   GCA ACG TTC ATC AAA CGT TTC CCT CAC AAT CCA CGC GAC TGG GAC AGG      2329
   Ala Thr Phe Ile Lys Arg Phe Pro His Asn Pro Arg Asp Trp Asp Arg
           720                 725                 730
   CTC CCC TAC CTC AAC CTA CCA TAC CTT CCA CCC AAT GCA GGA CGC CAG      2377
   Leu Pro Tyr Leu Asn Leu Pro Tyr Leu Pro Pro Asn Ala Gly Arg Gln
       735                 740                 745
   TAC CAC CTT GCC ATG GCT GCA TCA GAG TTC AAA GAG ACC CCC GAA CTC      2425
   Tyr His Leu Ala Met Ala Ala Ser Glu Phe Lys Glu Thr Pro Glu Leu
   750                 755                 760                 765
   GAG AGT GCC GTC AGA GCA ATG GAA GCA GCA GCC AAC GTG GAC CCA CTA          2473
   Glu Ser Ala Val Arg Ala Met Glu Ala Ala Ala Asn Val Asp Pro Leu
                   770                 775                 780
   TTC CAA TCT GCA CTC AGT GTG TTC ATG TGG CTG GAA GAG AAT GGG ATT          2521
   Phe Gln Ser Ala Leu Ser Val Phe Met Trp Leu Glu Glu Asn Gly Ile
               785                 790                 795
   GTG ACT GAC ATG GCC AAC TTC GCA CTC AGC GAC CCG AAC GCC CAT CGG          2569
   Val Thr Asp Met Ala Asn Phe Ala Leu Ser Asp Pro Asn Ala His Arg
           800                 805                 810
   ATG CGA AAT TTT CTT GCA AAC GCA CCA CAA GCA GGC AGC AAG TCG CAA          2617
   Met Arg Asn Phe Leu Ala Asn Ala Pro Gln Ala Gly Ser Lys Ser Gln
       815                 820                 825
   AGG GCC AAG TAC GGG ACA GCA GGC TAC GGA GTG GAG GCT CGG GGC CCC          2665
   Arg Ala Lys Tyr Gly Thr Ala Gly Tyr Gly Val Glu Ala Arg Gly Pro
   830                 835                 840                 845
   ACA CCA GAG GAA GCA CAG AGG GAA AAA GAC ACA CGG ATC TCA AAG AAG          2713
   Thr Pro Glu Glu Ala Gln Arg Glu Lys Asp Thr Arg Ile Ser Lys Lys
                   850                 855                 860
   ATG GAG ACC ATG GGC ATC TAC TTT GCA ACA CCA GAA TGG GTA GCA CTC          2761
   Met Glu Thr Met Gly Ile Tyr Phe Ala Thr Pro Glu Trp Val Ala Leu
               865                 870                 875
   AAT GGG CAC CGA GGG CCA AGC CCC GGC CAG CTA AAG TAC TGG CAG AAC          2809
   Asn Gly His Arg Gly Pro Ser Pro Gly Gln Leu Lys Tyr Trp Gln Asn
           880                 885                 890
   ACA CGA GAA ATA CCG GAC CCA AAC GAG GAC TAT CTA GAC TAC GTG CAT          2857
   Thr Arg Glu Ile Pro Asp Pro Asn Glu Asp Tyr Leu Asp Tyr Val His
       895                 900                 905
   GCA GAG AAG AGC CGG TTG GCA TCA GAA GAA CAA ATC CTA AGG GCA GCT          2905
   Ala Glu Lys Ser Arg Leu Ala Ser Glu Glu Gln Ile Leu Arg Ala Ala
   910                 915                 920                 925
   ACG TCG ATC TAC GGG GCT CCA GGA CAG GCA GAG CCA CCC CAA GCT TTC          2953
   Thr Ser Ile Tyr Gly Ala Pro Gly Gln Ala Glu Pro Pro Gln Ala Phe
                   930                 935                 940
   ATA GAC GAA GTT GCC AAA GTC TAT GAA ATC AAC CAT GGA CGT GGC CCA          3001
   Ile Asp Glu Val Ala Lys Val Tyr Glu Ile Asn His Gly Arg Gly Pro
               945                 950                 955
   AAC CAA GAA CAG ATG AAA GAT CTG CTC TTG ACT GCG ATG GAG ATG AAG      3049
   Asn Gln Glu Gln Met Lys Asp Leu Leu Leu Thr Ala Met Glu Met Lys
           960                 965                 970
   CAT CGC AAT CCC AGG CGG GCT CTA CCA AAG CCC AAG CCA AAA CCC AAT      3097
   His Arg Asn Pro Arg Arg Ala Leu Pro Lys Pro Lys Pro Lys Pro Asn
       975                 980                 985
   GCT CCA ACA CAG AGA CCC CCT GGT CGG CTG GGC CGC TGG ATC AGG ACC      3145
   Ala Pro Thr Gln Arg Pro Pro Gly Arg Leu Gly Arg Trp Ile Arg Thr
   990                 995                 1000                1005
   GTC TCT GAT GAG GAC CTT GAG TGAGGCTCCT GGGAGTCTCC CGACACCACC         3196
   Val Ser Asp Glu Asp Leu Glu
                   1010
   CGCGCAGGTG TGGACACCAA TTCGGCCTTA CAACATCCCA AATTGGATCC GTTCGCGGGT    3256
   CCCCT                                                                3261
(2) INFORMATION FOR SEQ ID NO: 6:
   (i) SEQUENCE CHARACTERISTICS:
      (A) LENGTH: 1012 amino acids
      (B) TYPE: amino acid
      (D) TOPOLOGY: linear
   (ii) MOLECULE TYPE: protein
   (xi) SEQUENCE DESCRIPTION: SEQ ID NO: 6:
   Met Thr Asn Leu Gln Asp Gln Thr Gln Gln Ile Val Pro Phe Ile Arg
    1               5                  10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Gly Asn Gly Asn Tyr
   65 70 75 80
   Lys Phe Asp Gln Met Leu Leu Thr Ala Gln Asn Leu Pro Ala Ser Tyr
   85 90 95
   Asn Tyr Cys Arg Leu Val Ser Arg Ser Leu Thr Val Arg Ser Ser Thr
   100 105 110
   Leu Pro Gly Gly Val Tyr Ala Leu Asn Gly Thr Ile Asn Ala Val Thr
   115 120 125
   Phe Gln Gly Ser Leu Ser Glu Leu Thr Asp Val Ser Tyr Asn Gly Leu
   130 135 140
   Met Ser Ala Thr Ala Asn Ile Asn Asp Lys Ile Gly Asn Val Leu Val
   145 150 155 160
   Gly Glu Gly Val Thr Val Leu Ser Leu Pro Thr Ser Tyr Asp Leu Gly
   165 170 175
   Tyr Val Arg Leu Gly Asp Pro Ile Pro Ala Ile Gly Leu Asp Pro Lys
   180 185 190
   Met Val Ala Thr Cys Asp Ser Ser Asp Arg Pro Arg Val Tyr Thr Ile
   195 200 205
   Thr Ala Ala Asp Asp Tyr Gln Phe Ser Ser Gln Tyr Gln Pro Gly Gly
   210 215 220
   Val Thr Ile Thr Leu Phe Ser Ala Asn Ile Asp Ala Ile Thr Ser Leu
   225 230 235 240
   Ser Val Gly Gly Glu Leu Val Phe Gln Thr Ser Val His Gly Leu Val
   245 250 255
   Leu Gly Ala Thr Ile Tyr Leu Ile Gly Phe Asp Gly Thr Thr Val Ile
   260 265 270
   Thr Arg Ala Val Ala Ala Asn Asn Gly Leu Thr Thr Gly Thr Asp Asn
   275 280 285
   Leu Met Pro Phe Asn Leu Val Ile Pro Thr Asn Glu Ile Thr Gln Pro
   290 295 300
   Ile Thr Ser Ile Lys Leu Glu Ile Val Thr Ser Lys Ser Gly Gly Gln
   305 310 315 320
   Ala Gly Asp Gln Met Ser Trp Ser Ala Arg Gly Ser Leu Ala Val Thr
   325 330 335
   Ile His Gly Gly Asn Tyr Pro Gly Ala Leu Arg Pro Val Thr Leu Val
   340 345 350
   Ala Tyr Glu Arg Val Ala Thr Gly Ser Val Val Thr Val Ala Gly Val
   355 360 365
   Ser Asn Phe Glu Leu Ile Pro Asn Pro Glu Leu Ala Lys Asn Leu Val
   370 375 380
   Thr Glu Tyr Gly Arg Phe Asp Pro Gly Ala Met Asn Tyr Thr Lys Leu
   385 390 395 400
   Ile Leu Ser Glu Arg Asp Arg Leu Gly Ile Lys Thr Val Trp Pro Thr
   405 410 415
   Arg Glu Tyr Thr Asp Phe Arg Glu Tyr Phe Met Glu Val Ala Asp Leu
   420 425 430
   Asn Ser Pro Leu Lys Ile Ala Gly Ala Phe Gly Phe Lys Asp Ile Ile
   435 440 445
   Arg Ala Ile Arg Arg Ile Ala Val Pro Val Val Ser Thr Leu Phe Pro
   450 455 460
   Pro Ala Ala Pro Leu Ala His Ala Ile Gly Glu Gly Val Asp Tyr Leu
   465 470 475 480
   Leu Gly Asp Glu Ala Gln Ala Ala Ser Gly Thr Ala Arg Ala Ala Ser
   485 490 495
   Gly Lys Ala Arg Ala Ala Ser Gly Arg Ile Arg Gln Leu Thr Leu Ala
   500 505 510
   Ala Asp Lys Gly Tyr Glu Val Val Ala Asn Leu Phe Gln Val Pro Gln
   515 520 525
   Asn Pro Val Val Asp Gly Ile Leu Ala Ser Pro Gly Val Leu Arg Gly
   530 535 540
   Ala His Asn Leu Asp Cys Val Leu Arg Glu Gly Ala Thr Leu Phe Pro
   545 550 555 560
   Val Val Ile Thr Thr Val Glu Asp Ala Met Thr Pro Lys Ala Leu Asn
   565 570 575
   Ser Lys Met Phe Ala Val Ile Glu Gly Val Arg Glu Asp Leu Gln Pro
   580 585 590
   Pro Ser Gln Arg Gly Ser Phe Ile Arg Thr Leu Ser Gly His Arg Val
   595 600 605
   Tyr Gly Tyr Ala Pro Asp Gly Val Leu Pro Leu Glu Thr Gly Arg Asp
   610 615 620
   Tyr Thr Val Val Pro Ile Asp Asp Val Trp Asp Asp Ser Ile Met Leu
   625 630 635 640
   Ser Lys Asp Pro Ile Pro Pro Ile Val Gly Asn Ser Gly Asn Leu Ala
   645 650 655
   Ile Ala Tyr Met Asp Val Phe Arg Pro Lys Val Pro Ile His Val Ala
   660 665 670
   Met Thr Gly Ala Leu Asn Ala Cys Gly Glu Ile Glu Lys Val Ser Phe
   675 680 685
   Arg Ser Thr Lys Leu Ala Thr Ala His Arg Leu Gly Leu Arg Leu Ala
   690 695 700
   Gly Pro Gly Ala Phe Asp Val Asn Thr Gly Pro Asn Trp Ala Thr Phe
   705 710 715 720
   Ile Lys Arg Phe Pro His Asn Pro Arg Asp Trp Asp Arg Leu Pro Tyr
   725 730 735
   Leu Asn Leu Pro Tyr Leu Pro Pro Asn Ala Gly Arg Gln Tyr His Leu
   740 745 750
   Ala Met Ala Ala Ser Glu Phe Lys Glu Thr Pro Glu Leu Glu Ser Ala
   755 760 765
   Val Arg Ala Met Glu Ala Ala Ala Asn Val Asp Pro Leu Phe Gln Ser
   770 775 780
   Ala Leu Ser Val Phe Met Trp Leu Glu Glu Asn Gly Ile Val Thr Asp
   785 790 795 800
   Met Ala Asn Phe Ala Leu Ser Asp Pro Asn Ala His Arg Met Arg Asn
   805 810 815
   Phe Leu Ala Asn Ala Pro Gln Ala Gly Ser Lys Ser Gln Arg Ala Lys
   820 825 830
   Tyr Gly Thr Ala Gly Tyr Gly Val Glu Ala Arg Gly Pro Thr Pro Glu
           835                 840                 845
   Glu Ala Gln Arg Glu Lys Asp Thr Arg Ile Ser Lys Lys Met Glu Thr
       850                 855                 860
   Met Gly Ile Tyr Phe Ala Thr Pro Glu Trp Val Ala Leu Asn Gly His
   865                 870                 875                 880
   Arg Gly Pro Ser Pro Gly Gln Leu Lys Tyr Trp Gln Asn Thr Arg Glu
                   885                 890                 895
   Ile Pro Asp Pro Asn Glu Asp Tyr Leu Asp Tyr Val His Ala Glu Lys
               900                 905                 910
   Ser Arg Leu Ala Ser Glu Glu Gln Ile Leu Arg Ala Ala Thr Ser Ile
           915                 920                 925
   Tyr Gly Ala Pro Gly Gln Ala Glu Pro Pro Gln Ala Phe Ile Asp Glu
       930                 935                 940
   Val Ala Lys Val Tyr Glu Ile Asn His Gly Arg Gly Pro Asn Gln Glu
   945                 950                 955                 960
   Gln Met Lys Asp Leu Leu Leu Thr Ala Met Glu Met Lys His Arg Asn
                   965                 970                 975
   Pro Arg Arg Ala Leu Pro Lys Pro Lys Pro Lys Pro Asn Ala Pro Thr
               980                 985                 990
   Gln Arg Pro Pro Gly Arg Leu Gly Arg Trp Ile Arg Thr Val Ser Asp
           995                 1000                1005
   Glu Asp Leu Glu
       1010
(2) INFORMATION FOR SEQ ID NO: 7:
   (i) SEQUENCE CHARACTERISTICS:
      (A) LENGTH: 3261 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION:97..531
   (xi) SEQUENCE DESCRIPTION: SEQ ID NO: 7:
(2) INFORMATION FOR SEQ ID NO: 8:
   (i) SEQUENCE CHARACTERISTICS:
      (A) LENGTH: 2827 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION:112..2745
   (xi) SEQUENCE DESCRIPTION: SEQ ID NO: 8:
   GGATACGATG GGTCTGACCC TCTGGGAGTC ACGAATTAAC GTGGCTACTA GGGGCGATAC        60
   CCGCCGCTGG CTGCCACGTT AGTGGCTCCT CTTCTTGATG ATTCTGCCAC C ATG AGT        117
                                                            Met Ser
                                                              1
   GAC ATT TTC AAC AGT CCA CAG GCG CGA AGC ACG ATC TCA GCA GCG TTC         165
   Asp Ile Phe Asn Ser Pro Gln Ala Arg Ser Thr Ile Ser Ala Ala Phe
             5                  10                  15
   GGC ATA AAG CCT ACT GCT GGA CAA GAC GTG GAA GAA CTC TTG ATC CCT         213
   Gly Ile Lys Pro Thr Ala Gly Gln Asp Val Glu Glu Leu Leu Ile Pro
        20                  25                  30
   AAA GTT TGG GTG CCA CCT GAG GAT CCG CTT GCC AGC CCT AGT CGA CTG        261
   Lys Val Trp Val Pro Pro Glu Asp Pro Leu Ala Ser Pro Ser Arg Leu
    35                  40                  45                  50
   GCA AAG TTC CTC AGA GAG AAC GGC TAC AAA GTT TTG CAG CCG CGG TCT        309
   Ala Lys Phe Leu Arg Glu Asn Gly Tyr Lys Val Leu Gln Pro Arg Ser
                    55                  60                  65
   CTG CCC GAG AAT GAG GAG TAT GAG ACC GAC CAA ATA CTC CCA GAC TTA        357
   Leu Pro Glu Asn Glu Glu Tyr Glu Thr Asp Gln Ile Leu Pro Asp Leu
                70                  75                  80
   GCA TGG ATG CGA CAG ATA GAA GGG GCT GTT TTA AAA CCC ACT CTA TCT        405
   Ala Trp Met Arg Gln Ile Glu Gly Ala Val Leu Lys Pro Thr Leu Ser
            85                  90                  95
   CTC CCT ATT GGA GAT CAG GAG TAC TTC CCA AAG TAC TAC CCA ACA CAT        453
   Leu Pro Ile Gly Asp Gln Glu Tyr Phe Pro Lys Tyr Tyr Pro Thr His
       100                 105                 110
   CGC CCT AGC AAG GAG AAG CCC AAT GCG TAC CCG CCA GAC ATC GCA CTA        501
   Arg Pro Ser Lys Glu Lys Pro Asn Ala Tyr Pro Pro Asp Ile Ala Leu
   115                 120                 125                 130
   CTC AAG CAG ATG ATT TAC CTG TTT CTC CAG GTT CCA GAG GCC AAC GAG        549
   Leu Lys Gln Met Ile Tyr Leu Phe Leu Gln Val Pro Glu Ala Asn Glu
                   135                 140                 145
   GGC CTA AAG GAT GAA GTA ACC CTC TTG ACC CAA AAC ATA AGG GAC AAG        597
   Gly Leu Lys Asp Glu Val Thr Leu Leu Thr Gln Asn Ile Arg Asp Lys
               150                 155                 160
   GCC TAT GGA AGT GGG ACC TAC ATG GGA CAA GCA ACT CGA CTT GTG GCC        645
   Ala Tyr Gly Ser Gly Thr Tyr Met Gly Gln Ala Thr Arg Leu Val Ala
           165                 170                 175
   ATG AAG GAG GTC GCC ACT GGA AGA AAC CCA AAC AAG GAT CCT CTA AAG        693
   Met Lys Glu Val Ala Thr Gly Arg Asn Pro Asn Lys Asp Pro Leu Lys
       180                 185                 190
   CTT GGG TAC ACT TTT GAG AGC ATC GCG CAG CTA CTT GAC ATC ACA CTA        741
   Leu Gly Tyr Thr Phe Glu Ser Ile Ala Gln Leu Leu Asp Ile Thr Leu
   195                 200                 205                 210
   CCG GTA GGC CCA CCC GGT GAG GAT GAC AAG CCC TGG GTG CCA CTC ACA        789
   Pro Val Gly Pro Pro Gly Glu Asp Asp Lys Pro Trp Val Pro Leu Thr
                   215                 220                 225
   AGA GTG CCG TCA CGG ATG TTG GTG CTG ACG GGA GAC GTA GAT GGC GAC      837
   Arg Val Pro Ser Arg Met Leu Val Leu Thr Gly Asp Val Asp Gly Asp
               230                 235                 240
   TTT GAG GTT GAA GAT TAC CTT CCC AAA ATC AAC CTC AAG TCA TCA AGT      885
   Phe Glu Val Glu Asp Tyr Leu Pro Lys Ile Asn Leu Lys Ser Ser Ser
           245                 250                 255
   GGA CTA CCA TAT GTA GGT CGC ACC AAA GGA GAG ACA ATT GGC GAG ATG      933
   Gly Leu Pro Tyr Val Gly Arg Thr Lys Gly Glu Thr Ile Gly Glu Met
       260                 265                 270
   ATA GCT ATA TCA AAC CAG TTT CTC AGA GAG CTA TCA ACA CTG TTG AAG      981
   Ile Ala Ile Ser Asn Gln Phe Leu Arg Glu Leu Ser Thr Leu Leu Lys
   275                 280                 285                 290
   CAA GGT GCA GGG ACA AAG GGG TCA AAC AAG AAG AAG CTA CTC AGC ATG     1029
   Gln Gly Ala Gly Thr Lys Gly Ser Asn Lys Lys Lys Leu Leu Ser Met
                   295                 300                 305
   TTA AGT GAC TAT TGG TAC TTA TCA TGC GGG CTT TTG TTT CCA AAG GCT     1077
   Leu Ser Asp Tyr Trp Tyr Leu Ser Cys Gly Leu Leu Phe Pro Lys Ala
               310                 315                 320
   GAA AGG TAC GAC AAA AGT ACA TGG CTC ACC AAG ACC CGG AAC ATA TGG     1125
   Glu Arg Tyr Asp Lys Ser Thr Trp Leu Thr Lys Thr Arg Asn Ile Trp
           325                 330                 335
   TCA GCT CCA TCC CCA ACA CAC CTC ATG ATC TCC ATG ATC ACC TGG CCC     1173
   Ser Ala Pro Ser Pro Thr His Leu Met Ile Ser Met Ile Thr Trp Pro
       340                 345                 350
   GTG ATG TCC AAC AGC CCA AAT AAC GTG TTG AAC ATT GAA GGG TGT CCA     1221
   Val Met Ser Asn Ser Pro Asn Asn Val Leu Asn Ile Glu Gly Cys Pro
   355                 360                 365                 370
   TCA CTC TAC AAA TTC AAC CCG TTC AGA GGA GGG TTG AAC AGG ATC GTC     1269
   Ser Leu Tyr Lys Phe Asn Pro Phe Arg Gly Gly Leu Asn Arg Ile Val
                   375                 380                 385
   GAG TGG ATA TTG GCC CCG GAA GAA CCC AAG GCT CTT GTA TAT GCG GAC     1317
   Glu Trp Ile Leu Ala Pro Glu Glu Pro Lys Ala Leu Val Tyr Ala Asp
               390                 395                 400
   AAC ATA TAC ATT GTC CAC TCA AAC ACG TGG TAC TCA ATT GAC CTA GAG     1365
   Asn Ile Tyr Ile Val His Ser Asn Thr Trp Tyr Ser Ile Asp Leu Glu
           405                 410                 415
   AAG GGT GAG GCA AAC TGC ACT CGC CAA CAC ATG CAA GCC GCA ATG TAC      1413
   Lys Gly Glu Ala Asn Cys Thr Arg Gln His Met Gln Ala Ala Met Tyr
       420                 425                 430
   TAC ATA CTC ACC AGA GGG TGG TCA GAC AAC GGC GAC CCA ATG TTC AAT      1461
   Tyr Ile Leu Thr Arg Gly Trp Ser Asp Asn Gly Asp Pro Met Phe Asn
   435                 440                 445                 450
   CAA ACA TGG GCC ACC TTT GCC ATG AAC ATT GCC CCT GCT CTA GTG GTG      1509
   Gln Thr Trp Ala Thr Phe Ala Met Asn Ile Ala Pro Ala Leu Val Val
                   455                 460                 465
   GAC TCA TCG TGC CTG ATA ATG AAC CTG CAA ATT AAG ACC TAT GGT CAA      1557
   Asp Ser Ser Cys Leu Ile Met Asn Leu Gln Ile Lys Thr Tyr Gly Gln
               470                 475                 480
   GGC AGC GGG AAT GCA GCC ACG TTC ATC AAC AAC CAC CTC TTG AGC ACG      1605
   Gly Ser Gly Asn Ala Ala Thr Phe Ile Asn Asn His Leu Leu Ser Thr
           485                 490                 495
   CTA GTG CTT GAC CAG TGG AAC TTG ATG AGA CAG CCC AGA CCA GAC AGC      1653
   Leu Val Leu Asp Gln Trp Asn Leu Met Arg Gln Pro Arg Pro Asp Ser
       500                 505                 510
   GAG GAG TTC AAA TCA ATT GAG GAC AAG CTA GGT ATC AAC TTT AAG ATT      1701
   Glu Glu Phe Lys Ser Ile Glu Asp Lys Leu Gly Ile Asn Phe Lys Ile
   515                 520                 525                 530
   GAG AGG TCC ATT GAT GAT ATC AGG GGC AAG CTG AGA CAG CTT GTC CTC      1749
   Glu Arg Ser Ile Asp Asp Ile Arg Gly Lys Leu Arg Gln Leu Val Leu
                   535                 540                 545
   CTT GCA CAA CCA GGG TAC CTG AGT GGG GGG GTT GAA CCA GAA CAA TCC      1797
   Leu Ala Gln Pro Gly Tyr Leu Ser Gly Gly Val Glu Pro Glu Gln Ser
               550                 555                 560
   AGC CCA ACT GTT GAG CTT GAC CTA CTA GGG TGG TCA GCT ACA TAC AGC      1845
   Ser Pro Thr Val Glu Leu Asp Leu Leu Gly Trp Ser Ala Thr Tyr Ser
           565                 570                 575
   AAA GAT CTC GGG ATC TAT GTG CCG GTG CTT GAC AAG GAA CGC CTA TTT      1893
   Lys Asp Leu Gly Ile Tyr Val Pro Val Leu Asp Lys Glu Arg Leu Phe
       580                 585                 590
   TGT TCT GCT GCG TAT CCC AAG GGA GTA GAG AAC AAG AGT CTC AAG TCC      1941
   Cys Ser Ala Ala Tyr Pro Lys Gly Val Glu Asn Lys Ser Leu Lys Ser
   595                 600                 605                 610
   AAA GTC GGG ATC GAG CAG GCA TAC AAG GTA GTC AGG TAT GAG GCG TTG      1989
   Lys Val Gly Ile Glu Gln Ala Tyr Lys Val Val Arg Tyr Glu Ala Leu
                   615                 620                 625
   AGG TTG GTA GGT GGT TGG AAC TAC CCA CTC CTG AAC AAA GCC TGC AAG      2037
   Arg Leu Val Gly Gly Trp Asn Tyr Pro Leu Leu Asn Lys Ala Cys Lys
               630                 635                 640
   AAT AAC GCA GGC GCC GCT CGG CGG CAT CTG GAG GCC AAG GGG TTC CCA      2085
   Asn Asn Ala Gly Ala Ala Arg Arg His Leu Glu Ala Lys Gly Phe Pro
           645                 650                 655
   CTC GAC GAG TTC CTA GCC GAG TGG TCT GAG CTG TCA GAG TTC GGT GAG      2133
   Leu Asp Glu Phe Leu Ala Glu Trp Ser Glu Leu Ser Glu Phe Gly Glu
       660                 665                 670
   GCC TTC GAA GGC TTC AAT ATC AAG CTG ACC GTA ACA TCT GAG AGC CTA      2181
   Ala Phe Glu Gly Phe Asn Ile Lys Leu Thr Val Thr Ser Glu Ser Leu
   675                 680                 685                 690
   GCC GAA CTG AAC AAG CCA GTA CCC CCC AAG CCC CCA AAT GTC AAC AGA      2229
   Ala Glu Leu Asn Lys Pro Val Pro Pro Lys Pro Pro Asn Val Asn Arg
                   695                 700                 705
   CCA GTC AAC ACT GGG GGA CTC AAG GCA GTC AGC AAC GCC CTC AAG ACC      2277
   Pro Val Asn Thr Gly Gly Leu Lys Ala Val Ser Asn Ala Leu Lys Thr
               710                 715                 720
   GGT CGG TAC AGG AAC GAA GCC GGA CTG AGT GGT CTC GTC CTT CTA GCC      2325
   Gly Arg Tyr Arg Asn Glu Ala Gly Leu Ser Gly Leu Val Leu Leu Ala
           725                 730                 735
   ACA GCA AGA AGC CGT CTG CAA GAT GCA GTT AAG GCC AAG GCA GAA GCC      2373
   Thr Ala Arg Ser Arg Leu Gln Asp Ala Val Lys Ala Lys Ala Glu Ala
       740                 745                 750
   GAG AAA CTC CAC AAG TCC AAG CCA GAC GAC CCC GAT GCA GAC TGG TTC      2421
   Glu Lys Leu His Lys Ser Lys Pro Asp Asp Pro Asp Ala Asp Trp Phe
   755                 760                 765                 770
   GAA AGA TCA GAA ACT CTG TCA GAC CTT CTG GAG AAA GCC GAC ATC GCC      2469
   Glu Arg Ser Glu Thr Leu Ser Asp Leu Leu Glu Lys Ala Asp Ile Ala
                   775                 780                 785
   AGC AAG GTC GCC CAC TCA GCA CTC GTG GAA ACA AGC GAC GCC CTT GAA      2517
   Ser Lys Val Ala His Ser Ala Leu Val Glu Thr Ser Asp Ala Leu Glu
               790                 795                 800
   GCA GTT CAG TCG ACT TCC GTG TAC ACC CCC AAG TAC CCA GAA GTC AAG      2565
   Ala Val Gln Ser Thr Ser Val Tyr Thr Pro Lys Tyr Pro Glu Val Lys
           805                 810                 815
   AAC CCA CAG ACC GCC TCC AAC CCC GTT GTT GGG CTC CAC CTG CCC GCC      2613
   Asn Pro Gln Thr Ala Ser Asn Pro Val Val Gly Leu His Leu Pro Ala
       820                 825                 830
   AAG AGA GCC ACC GGT GTC CAG GCC GCT CTT CTC GGA GCA GGA ACG AGC      2661
   Lys Arg Ala Thr Gly Val Gln Ala Ala Leu Leu Gly Ala Gly Thr Ser
   835                 840                 845                 850
   AGA CCA ATG GGG ATG GAG GCC CCA ACA CGG TCC AAG AAC GCC GTG AAA      2709
   Arg Pro Met Gly Met Glu Ala Pro Thr Arg Ser Lys Asn Ala Val Lys
                   855                 860                 865
   ATG GCC AAA CGG CGG CAA CGC CAA AAG GAG AGC CGC TAACAGCCAT           2755
   Met Ala Lys Arg Arg Gln Arg Gln Lys Glu Ser Arg
               870                 875
   GATGGGAACC ACTCAAGAAG AGGACACTAA TCCCAGACCC CGTATCCCCG GCCTTCGCCT    2815
   GCGGGGGCCC CC                                                        2827

## Description

The present invention is concerned with a bimavirus mutant, a vaccine comprising this mutant, a method for determining birnavirus infection in an animal, as well as with a test kit for carrying out this method.

Infectious bursal disease virus (IBDV) and Infectious pancreatic necrosis virus (IPNV) are members of the Birnaviridae family. Viruses in this family have a very similar genomic organisation and a similar replication cycle. The genomes of these viruses consist of 2 segments (A and B) of double-stranded (ds) RNA. The larger segment A encodes a polyprotein which is cleaved by autoproteolysis to form mature viral proteins VP2, VP3 and VP4 (Hudson, P.J. et al, Nucleic Acids Res., 14, 5001-50012, 1986; Dobos P., Annual review of fish diseases 5, 25-54, 1995). VP2 and VP3 are the major structural proteins of the virion. VP2 is the major host-protective immunogen of birnaviruses, and contains the antigenic regions responsible for the induction of neutralising antibodies. The VP4 protein appears to be a virus-coded protease that is involved in the processing of a precursor polyprotein of the VP2, VP3 and VP4 proteins. The larger segment A possesses also a second open reading frame (ORF), preceding and partially overlapping the polyprotein gene. This second open reading frame encodes a protein VP5 of unknown function that is present in IBDV infected cells (Mundt, E. et al., J. Gen. Virol., 76, 437-443, 1995).

The smaller segment B encodes VP1, a 90 kDa multifunctional protein with polymerase and capping enzyme activities (Spies, U. et al., Virus Res., 8, 127-140, 1987 and Spies, U. et al., J. Gen. Virol., 71, 977-981, 1990; Duncan R. et al., Virology 181, 541-552, 1991).

For IBDV, two serotypes exist, serotype 1 and 2. The 2 serotypes may be differentiated by virus neutralisation (VN) tests. Furthermore, subtypes of serotype 1 have been isolated. These so-called "variant" viruses of serotype 1 can be identified by cross-neutralisation tests (Diseases of Poultry, 9th edition, 1991, Wolfe Publishing Ltd, ISBN 0 7234 1706 7, Chapter 28, P.D. Lukert and Y.M. Saif, 648-663), a panel of monoclonal antibodies (Snyder, D.B. et al., Arch. Virol., 127, 89-101. 1992.) or RT-PCR (Jackwood, D.J., Proceedings of the International symposium on infectious bursal disease and chicken infectious anaemia, Rauischholzhausen, Germany, 155-161, 1994). Some of these subtypes of serotype 1 of IBDV have been described in literature for example: Classical, Variant-E, GLS, RS593 and DS326 strains (Van Loon, et al. Proceedings of the International symposium on infectious bursal disease and chicken infectious anaemia, Rauischholzhausen, Germany, 179-187, 1994).

Infectious Bursal disease (IBD), also called Gumboro disease, is an acute, highly-contagious viral infection in chickens that has lymphoid tissue as its primary target with a selective tropism for cells of the bursa of Fabricius. The morbidity rate in susceptible flocks is high, with rapid weight loss and moderate mortality rates. Chicks that recover from the disease may have immune deficiencies because of the destruction of the bursa of Fabricius which is essential to the defence mechanism of the chicken. The IBD-virus causes severe immunosuppression in chickens younger than 3 weeks of age and induces bursal lesions in chicks up to 3 months old.

For many years the disease could be prevented by inducing high levels of antibodies in breeder flocks by the application of an inactivated vaccine, to chickens that had been primed with attenuated live IBDV vaccine. This has kept economic losses caused by IBD to a minimum. Maternal antibodies in chickens derived from vaccinated breeders prevents early infection with IBDV and diminishes problems associated with immunosuppression. In addition, attenuated live vaccines have also been used successfully in commercial chicken flocks after maternal antibodies had declined.

Recently, very virulent strains of IBDV have caused outbreaks of disease with high mortality in Europe. The current vaccination programs failed to protect chicks sufficiently. Vaccination failures were mainly due to the inability of live vaccines to infect the birds before challenge with virulent field virus.

Eradication of the disease by other preventative measures than vaccination has not been feasible, because the virus is widely spread and because with currently administered live attenuated or inactivated IBDV vaccines it is not possible to determine whether a specific animal is infected with an IBDV field virus or whether the animal was vaccinated with an IBDV vaccine. In order to be able to start an eradication control programme for IBDV it is highly desirable that the possibility exists to discriminate between animals vaccinated with an IBDV vaccine and those infected with a field virus so as to be able to take appropriate measures, i.e. remove infected flocks, to reduce spreading of the virulent field virus. The introduction of, for example, a serologically identifiable marker can be achieved by introducing a mutation in genes encoding non-essential (glyco)proteins of the IBDV which still give rise to the production of antibodies in an infected host animal. A marker vaccine for Aujeszky's disease and companion diagnostic tests have proven their practical value in the control of this disease. Whereas such control programs for other viral infectious diseases in animals are under development, until the present invention a vaccine based on an IBDV vaccine strain which would fit in IBDV control programs has not been described yet.

Mundt and Köllner (Annual Meeting of the German Federal Research Institute for virus diseases in animals, March 1997)) disclose the construction of an IBDV mutant that fails to express a VP5 protein as a result of a genetically engineered mutation in the start codon of the VP5 gene. This mutation comprises the substitution of one nucleotide in the start codon of the VP5 gene.

The present invention provides a birnavirus mutant as defined in the claims which is not able to produce a native VP5 protein as a result of a mutation in the VP5 gene of the birnavirus genome.

Preferably, the birnavirus mutant is an IBDV mutant or an IPNV mutant, the IBDV mutant being most preferred, in particular an IBDV mutant derived from a serotype 1 IBD virus is provided by the present invention.

It is demonstrated that an IBDV mutant that is not able to produce a VP5 protein is still able to infect poultry and to replicate in the infected host animals in vivo, i.e. evidence is provided that the gene encoding the VP5 protein is a non-essential gene. Example 3 and 4 show that VP5⁻ IBDV can be re-isolated from organs of animals infected with the IBDV mutant and that the IBDV mutant induces a protective immune response in the infected animals.

Moreover, it has been established herein that part of the normal anti-IBDV immune response in poultry is directed to the VP5 region. This is rather surprising as the VP5 protein is considered to represent a non-structural viral protein (Mundt et al., J. Gen. Virol. 76, 437-443, 1995) and the immune response in an animal against a viral pathogen is usually elicited against the structural (glyco)proteins of the virus. These findings make the IBDV mutant and other birnavirus mutants according to the present invention a suitable vaccine candidate for a marker vaccine. Such a marker vaccine provides the possibility to determine whether animals are infected with a wild-type birnavirus, e.g. IBDV, or with a vaccine virus.

Additionally, it has been found that the VP5 protein is involved in the expression of virulence of the birnaviruses, in particular of IBDV, and that the inability of the virus mutants to produce the native VP5 protein leads to an attenuation of the virus.

With the term "which is not able to produce a native VP5 protein" is meant that the birnavirus mutant produces a polypeptide that can be distinguished by serological tests from the native VP5 protein, or does not produce a VP5 protein at all. For example, in the former case, the birnavirus mutant produces only a fragment of the native birnavirus VP5 protein which lacks one or more immunogenic epitopes.

Preferably, the birnavirus mutant according to the invention produces no VP5 protein upon infection of a host cell.

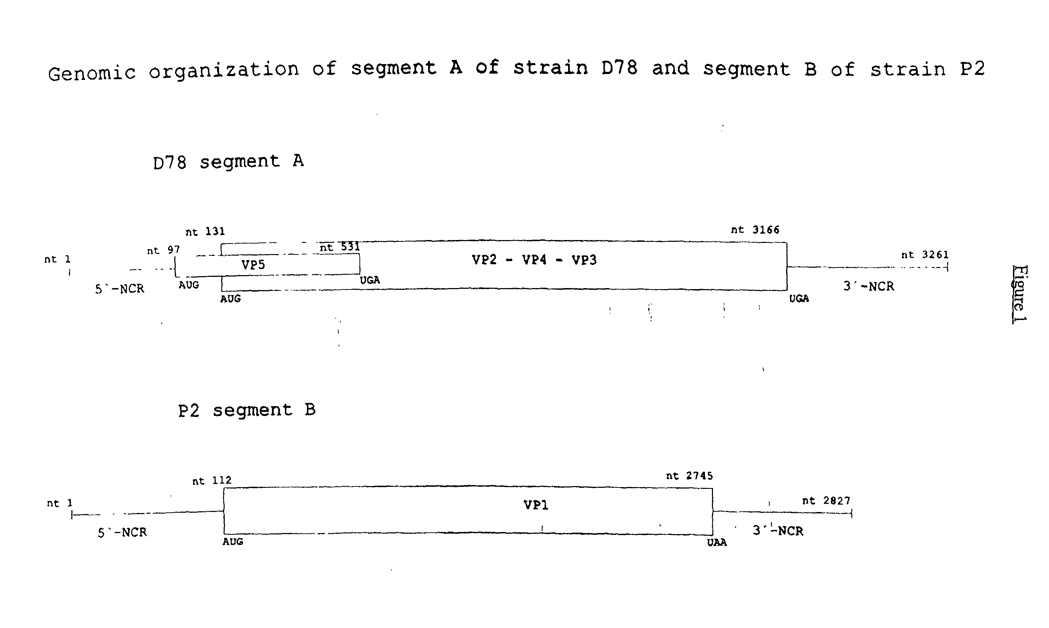

As described above, the genomic organisation of the birnaviruses is well established: the IBDV and IPNV genome comprises a large segment A and a smaller segment B. The segment A of IBDV comprises a large open reading frame (ORF) encoding a polyprotein of about 110 kDa (VP2-VP4-VP3). The gene encoding the VP5 protein is identified in the prior art, and defined herein, as the small ORF on segment A of the birnavirus genome which precedes and partially overlaps the polyprotein encoding ORF (Bayliss et al., J. Gen. Virol. 71, 1303-1312, 1990; Spies et al., J. Gen. Virol. 71, 977-981, 1990; Havarstein L.S. et al., J. Gen. Virology 71, 299-308; 1990; Dobos et al., 1995, supra; Figures 1-3 herein and SEQ ID No.'s 1-7). The mutation introduced in the VP5 gene is such that it does not prevent the expression of the polyprotein.

SEQ ID No. 1 comprises the full length cDNA nucleotide sequence of segment B of IBDV strain P2, as well as the amino acid sequence of the VP1 protein encoded by segment B (see also SEQ ID. No. 2). SEQ ID No. 3 and 5 depict the full length cDNA sequence of segment A of IBDV strain D78 and the coding region of the VP5 protein and the polyprotein, respectively. SEQ ID 3 and 4 also show the amino acid sequence of the D78 VP5 protein. SEQ ID No. 5 and 6 show the amino acid sequence of the polyprotein VP2-VP4-VP3 of D78. SEQ ID No. 7 shows the 5'-end of segment A of strain D78, including the mutations introduced in the VP5 coding region. SEQ ID No. 8 shows the nucleotide sequence of segment B of strain D78 and the amino acid sequence of the D78 VP1 protein. The genomic organisation of both segments is also shown in Figure 1.

The ORF coding for VP5 is conserved in all hitherto published segment A sequences. The IBDV ORF encodes 145 amino acids resulting in a calculated molecular mass of 16.5 kDa. The nucleotide sequence of the ORF encoding the VP5 protein of IBDV strain D78 used herein is shown in SEQ ID No. 3 and 4. Natural variations may exist between individual IBDV isolates. These natural variations result from small differences in the genomes of these viruses. The nucleotide sequence of the segment A, including the nucleotide sequence of the VP5 gene for many IBDV isolates have been described in the prior art (Vakharia et al., Avian Diseases 36, 736-742, 1992; Bayliss et al., J. Gen. Virol. 71, 1303-1314, 1990; Hudson et al., Nuc. Acid Res. 14, 5001-5012, 1986; Schnitzler et al., J. Gen. Virol. 47, 1563-1571, 1993; Kibenge et al., J. Gen. Virol. 71, 569-577, 1990 and Virology 184, 437-440, 1991; Mundt et al., Virology 209, 10-18, 1995; Lana et al., Virus Genes 6, 247-259, 1992; Vakharia et al., Virus Res. 31, 265-273, 1994; Brown et al., Virus Res. 40, 1-15, 1996). The amino acid sequence of the VP5 protein from serotype I IBDV strains display a homology of at least 95% with the VP5 amino acid sequence shown in SEQ ID No. 3 and 4, whereas the homology between serotype II VP5 sequence and the amino acid sequence shown in SEQ ID No. 3 and 4 is at least 75%. Therefore, a preferred IBDV mutant according to the present invention is an IBDV mutant wherein the mutation is introduced in the VP5 gene having a homology of at least 75%, in particular at least 95% on the amino acid sequence level with the VP5 amino acid sequence shown herein.

Preferably an IBDV mutant according to the present invention is derived from any of the classical or variant (e.g. variant E or GLS) IBDV vaccine strains, such as those currently used in the field. Such suitable IBDV strains include the IBDV vaccine strains present in the commercially available vaccines: D78, PBG 98, LZ 228E, 89-03 (Intervet International B.V.), Bursine 2 (Fort Dodge Animal Health) and S 706 (Rhone Mérieux).

A particular preferred IBDV mutant according to the invention is derived from the D78 strain comprising a VP5 gene encoding a protein having the amino acid sequence shown in SEQ ID No. 3 and 4.

Alternatively, the parent birnavirus strain for the virus mutant according to the invention is a virulent birnavirus field strain. It is found herein that the VP5 protein is a factor associated with virulence, and that the absence of the native VP5 protein in a birnavirus results in an attenuated form of the virus.

Preferably the invention provides a birnavirus mutant which is not able to produce a native VP5 protein as a result of a mutation in the part of the VP5 gene which does not overlap with the large ORF encoding the polyprotein.

In particular, the birnavirus mutant according to the invention comprises a mutation in the 5'-end of the VP5 gene spanning nucleotides 1-30, preferably 1-20, more preferably 1-10. Most preferred is an birnavirus mutant having a mutation in nucleotides 1-3 of the VP5 gene.

A mutation is understood to be a change of the genetic information in the VP5 gene with respect to the genetic information present in this region of the genome of naturally occurring birnavirus producing native VP5 protein. The mutation is, for example, a nucleic acid substitution, deletion, insertion or inversion, or a combination thereof.

In a preferred embodiment of the present invention a birnavirus mutant is provided wherein the mutation is a substitution of one or more nucleotides. In particular, a nucleic acid substitution is introduced in the start codon, as a result of which the new codon encodes an amino acid different from methionine or represents a stop codon, preferably the nucleic acid substitution comprises at least two of the nucleotides of the start codon.

A further birnavirus mutant according to the invention comprises a substitution of one or more nucleotides in a codon(s) different from the start codon resulting in one or more stop codons, preferably in the 5'-end of the VP5 gene as defined above, if desired in addition to a substitution in the start codon as described above. Preferably, the birnavirus mutant comprises a stop codon in this region of the VP5 gene in each of the three reading frames.

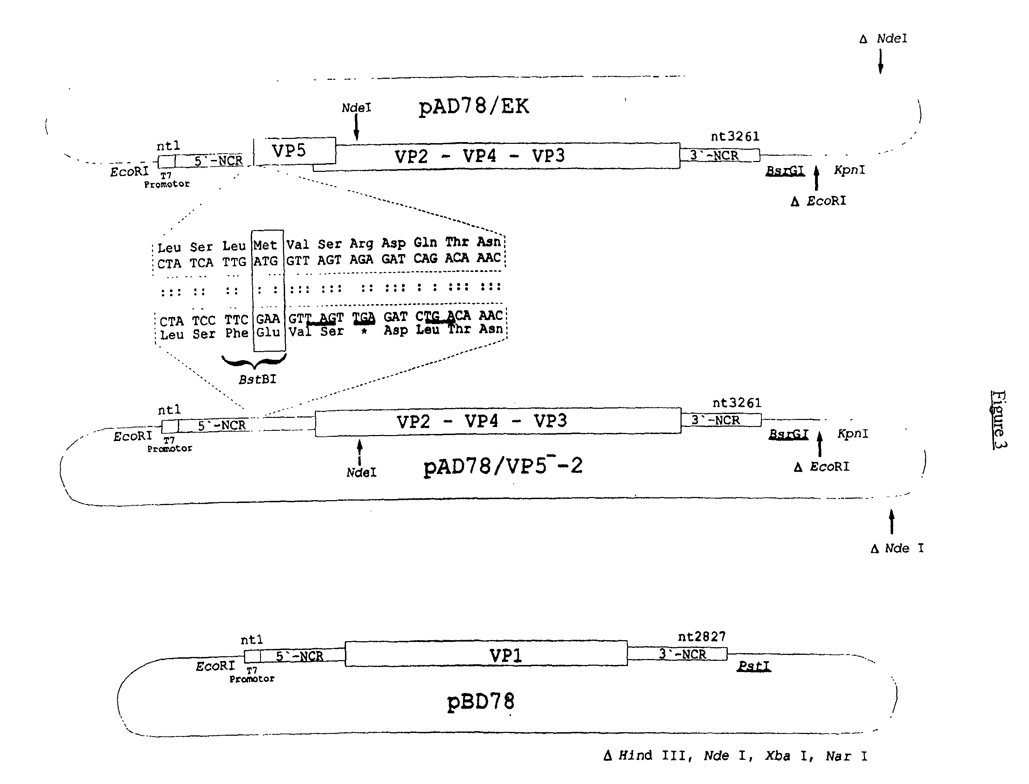

Such a preferred birnavirus mutant may be an IBDV mutant having a mutation in the start codon, the fourth and the sixth codon of the VP5 gene, preferably resulting in the mutated codons shown in SEQ ID No. 7 and Figure 3.

Alternatively, a birnavirus mutant is provided wherein the mutation is a deletion. In particular, the deletion comprises less than 20, less than 10 or less than 5 nucleotides. Preferably, the deletion comprises a total number of nucleotides not dividable by three, resulting in a shift of the reading frame.

Preferably the deletion comprises one or more nucleotides of the start codon of the VP5 gene.

In an alternative embodiment of the present invention a bimavirus mutant is provided wherein the mutation comprises the insertion of a heterologous nucleic acid sequence in the birnavirus genome. A heterologous nucleic acid sequence is a nucleic acid sequence normally not present at the specific insertion site of the particular virus species.

The heterologous nucleic sequence to be incorporated into the birnavirus genome is a nucleic acid fragment which either encodes a polypeptide or is a non-coding sequence. The nucleic acid fragment can be derived from any source, e.g. viral, eukaryotic, prokaryotic or synthetic, including oligonucleotides suitable for the interruption of the expression of the VP5 gene.

A suitable oligonucleotide for the interruption of the VP5 expression may comprise three translational stop codons in each of the possible reading frames in both directions, in addition to one or more appropriate restriction enzyme cleavage sites useful for the insertion of a second heterologous nucleic acid sequence. The length and nucleotide sequence of such a non-coding heterologous nucleic acid sequence is not critical, but preferably varies between 8-50 nucleotides.

In a further embodiment of the present invention a bimavirus mutant is provided which can be used not only for the preparation of a vaccine against infection by a specific birnavirus, but also against other poultry or fish infectious diseases. For example, a vector vaccine based on such an IBDV mutant offers the possibility to immunise against other avian pathogens by the expression of antigens of these avian pathogens within infected cells of the immunised host. Such an IBDV vector according to the present invention can be obtained by inserting a heterologous nucleic acid sequence encoding a polypeptide heterologous to the IBDV in the VP5 gene as defined herein.

The heterologous nucleic acid sequence may encode an antigen of an avian pathogen such as Newcastle disease virus, Infectious bronchitis virus, Marek's disease virus, avian encephalomyelitis virus, avian reovirus, avian influenza virus, chicken anaemia virus, *Salmonella spp., E.coli, and Eimeria* spp.

Furthermore, an IBDV mutant according to the invention comprises in addition to the mutation in the VP5 gene, a mutation in the VP2 gene, wherein this gene expresses a chimeric protein comprising neutralising epitopes of more than one antigenic type of IBDV (e.g. classic, Variant-E and/or GLS). Preferably, such a mutant comprises the relevant protective VP2 epitopes of a variant GLS strain and classic strain. In particular, the mutated VP2 gene is a GLS VP2 gene comprising a nucleic acid sequence fragment encoding the B69 epitope. The construction of such a mutated VP2 genes is described in Snyder et al., Avian Diseases 38, 701-707, 1994. Furthermore, nucleic acid sequences encoding polypeptides for pharmaceutical or diagnostic applications, in particular immuno-modulators such as lymphokines, interferons or cytokines, may be incorporated into the VP5 gene. The heterologous nucleic acid sequence may also encode a screenable marker, such as E. coli β-galactosidase or E. coli β-glucuronidase.

The construction of birnavirus mutants, in particular of IBDV mutants according to the present invention can be achieved by means of the recently established infectious cRNA system for IBDV (Mundt and Vakharia, Proc. Natl. Acad. Sci. USA 93, 11131-11136, 1996). This reverse genetics system opens the possibility to introduce mutations in the RNA genome of the IBD virus, in particular in the VP5 gene. The most important step in this reverse genetics system is to provide full length cDNA clones of the segments A and B of IBD virus. cDNA constructs comprising the segment A or B, including the nucleotides of the 5'- and 3'- ends of both these segments can be generated according to the method described by Mundt and Vakharia (1996, supra). Additionally, these constructs comprise a RNA polymerase promoter operably linked to either of the segments. The promoter can be the promoter for the T7, SP6 or T3 polymerase, the T7 promoter being preferred. Mutations can be introduced into the VP5 gene by means of methods generally known in the art for this purpose. In particular, the mutation(s) are introduced by means of site directed mutagenesis.

For example, in a first step a cDNA fragment is provided comprising at least a substantial part of the VP5 gene. In the next step suitable primer pairs are designed and hybridised with the VP5 sequence containing fragment. The 5'-primer comprises in addition to sequences complementary to the VP5 sequence, nucleotides which harbour the desired mutation, e.g. a mutation which changes the ATG start codon to an AGG (arginine) codon. Moreover, the 5'-primer is provided with an upstream nucleotide sequence representing a suitable restriction enzyme cleavage site which allows the restoring of the complete 5'-end non-coding sequence. Subsequently, the new mutated fragment is amplified using PCR and the new fragment is introduced in the starting sequence by replacing the native nucleic acid sequence using appropriate restriction enzymes. In the next step plus-sense transcripts of the segment A and B are generated in vitro with (T7) RNA polymerease, after which the synthetic transcripts are purified using conventional RNA purification techniques. The recombinant IBDV mutant according to the invention is obtained after transfection of suitable cells (e.g. VERO cells, QM-7 cells or CEC cells) with the synthetic RNA transcripts of both segments of the IBDV genome, if desired in the presence of transfection-enhancing compositions, such as Lipofectin. Finally the recombinant IBDV is harvested from the supernatant of the transformed cells.

Methods for introducing a mutation in the birnavirus genome are described herein, but are also generally used in the art (Mundt and Vakharia, 1996, supra; Current Protocols in Molecular Biology, eds.: F. M. Ausubel et al., Wiley N.Y., 1995 edition, pages 8.5.1.-8.5.9.)

Further to the unexpected finding by the present inventors that the VP5 ORF of IBDV is a non-essential region of the IBDV genome, it has also been found that an IBDV mutant according to the present invention is able to induce a protective immune response, i.e. animals immunised with a vaccine comprising the IBDV mutant are protected against virulent challenge. Moreover, it has been found that anti-sera of animals infected with naturally occurring IBDV comprise antibodies directed to the non-structural VP5 protein and that these antisera can be distinguished from anti-sera derived from animals infected with an IBDV mutant according to the present invention. In addition, it has been found that the IBDV mutant as described above is attenuated if compared with the parent IBD virus which is able to produce the native VP5 protein.

Therefore, another aspect of this invention is a vaccine for use in the protection of animals against birnavirus infection comprising the birnavirus mutant as characterised above, together with a pharmaceutical acceptable carrier or diluent. In particular, the vaccine according to the invention is a vaccine for use in the protection of poultry against infectious bursal disease comprising the IBDV mutant described above.

The birnavirus mutant according to the present invention can be incorporated into the vaccine as live or inactivated virus.

A vaccine according to the invention can be prepared by conventional methods such as for example commonly used for the commercially available live- and inactivated IBDV vaccines. Briefly, a susceptible substrate is inoculated with an IBDV mutant according to the invention and propagated until the virus replicated to a desired infectious titre after which IBDV containing material is harvested.

Every substrate which is able to support the replication of IBD viruses can be used in the present invention, including primary (avian) cell cultures, such as chicken embryo fibroblast cells (CEF) or chicken kidney cells (CK), mammalian cell lines such as the VERO cell line or the BGM-70 cell line, or avian cell lines such as QT-35, QM-7 or LMH. Usually, after inoculation of the cells, the virus is propagated for 3-10 days, after which the cell culture supernatant is harvested, and if desired filtered or centrifuged in order to remove cell debris.

Alternatively, the IBDV mutant is propagated in embryonated chicken eggs. In particular, the substrate on which these IBD viruses are propagated are SPF embryonated eggs. Embryonated eggs can be inoculated with, for example 0.2 ml IBDV mutant containing suspension or homogenate comprising at least 10² TCID₅₀ per egg, and subsequently incubated at 37 °C. After about 2-5 days the IBD virus product can be harvested by collecting the embryo's and/or the membranes and/or the allantoic fluid followed by appropriate homogenising of this material. The homogenate can be centrifuged thereafter for 10 min at 2500 x g followed by filtering the supernatant through a filter (100 µm).

The vaccine according to the invention containing the live virus can be prepared and marketed in the form of a suspension or in a lyophilised form and additionally contains a pharmaceutically acceptable carrier or diluent customary used for such compositions. Carriers include stabilisers, preservatives and buffers. Suitable stabilisers are, for example SPGA, carbohydrates (such as sorbitol, mannitol, starch, sucrose, dextran, glutamate or glucose), proteins (such as dried milk serum, albumin or casein) or degradation products thereof. Suitable buffers are for example alkali metal phosphates. Suitable preservatives are thimerosal, merthiolate and gentamicin. Diluents include water, aqueous buffer (such as buffered saline), alcohols and polyols (such as glycerol).

If desired, the live vaccines according to the invention may contain an adjuvant. Examples of suitable compounds and compositions with adjuvant activity are the same as mentioned below.

Although administration by injection, e.g. intramuscular, subcutaneous of the live vaccine according to the present invention is possible, the vaccine is preferably administered by the inexpensive mass application techniques commonly used for IBDV vaccination. For IBDV vaccination these techniques include drinking water and spray vaccination.

Alternative methods for the administration of the live vaccine include in ovo, eye drop and beak dipping administration.

In another aspect of the present invention a vaccine is provided comprising the birnavirus mutant in an inactivated form. The major advantage of an inactivated vaccine is the extremely high levels of protective antibodies of long duration that can be achieved.

The aim of inactivation of the viruses harvested after the propagation step is to eliminate reproduction of the viruses. In general, this can be achieved by chemical or physical means. Chemical inactivation can be effected by treating the viruses with, for example, enzymes, formaldehyde, β-propiolactone, ethylene-imine or a derivative thereof. If necessary, the inactivating compound is neutralised afterwards. Material inactivated with formaldehyde can, for example, be neutralised with thiosulphate. Physical inactivation can preferably be carried out by subjecting the viruses to energy-rich radiation, such as UV light or γ-rays. If desired, after treatment the pH can be adjusted to a value of about 7.

A vaccine containing the inactivated birnavirus mutant can, for example comprise one or more of the above-mentioned pharmaceutically acceptable carriers or diluents suited for this purpose.

Preferably, an inactivated vaccine according to the invention comprises one or more compounds with adjuvant activity. Suitable compounds or compositions for this purpose include aluminium hydroxide, -phosphate or -oxide, oil-in-water or water-in-oil emulsion based on, for example a mineral oil, such as Bayol F® or Marcol 52® or a vegetable oil such as vitamin E acetate, and saponins.

The vaccine according to the invention comprises an effective dosage of the birnavirus mutant as the active component, i.e. an amount of immunising birnavirus material that will induce immunity in the vaccinated birds against challenge by a virulent virus. Immunity is defined herein as the induction of a significant higher level of protection in a population of birds after vaccination compared to an unvaccinated group.

Typically, the live vaccine according to the invention can be administered in a dose of 10²-10⁹ TCID₅₀ infectious dose₅₀ (TCID₅₀) per animal, preferably in a dose ranging from 10^{5.0}-10^{7.0} TCID₅₀, and an inactivated vaccines may contain the antigenic equivalent of 10⁵-10⁹ TCID₅₀ per animal.

Inactivated vaccines are usually administered parenterally, e.g. intramuscularly or subcutaneously.

Although, the IBDV vaccine according to the present invention may be used effectively in chickens, also other poultry such as turkeys, guinea fowl and partridges may be successfully vaccinated with the vaccine. Chickens include broilers, reproduction stock and laying stock.

The age of the animals receiving a live or inactivated vaccine according to the invention is the same as that of the animals receiving the conventional live- or inactivated IBDV vaccines. For example, broilers (free of maternally derived antibodies-MDA) may be vaccinated at one-day-old, whereas broilers with high levels of MDA are preferably vaccinated at 2-3 weeks of age. Laying stock or reproduction stock with low levels of MDA may be vaccinated at 1-10 days of age followed by booster vaccinations with inactivated vaccine on 6-8 and 16-20 weeks of age.

The invention also includes combination vaccines comprising, in addition to the IBDV or IPNV mutant according to the invention, one or more immunogens derived from other pathogens infectious to poultry or fish, respectively.

Preferably, the combination vaccine additionally comprises one or more vaccine strains of infectious bronchitis virus (IBV), Newcastle disease virus (NDV), egg drop syndrome (EDS) virus, turkey rhinotracheitis virus (TRTV) or reovirus.

In addition to a marker vaccine for birnaviruses, the availability of an appropriate diagnostic test is an essential requirement for the application of a birnavirus eradication control programme. Such a diagnostic test is provided herein and comprises a method for determining IBDV infection in poultry and IPNV infection in fish, i.e. it provides a method for distinguishing an animal in the field vaccinated with a vaccine as described above, from an animal infected with a naturally-occurring IBDV or IPNV.

Therefore, the present invention provides a method for the detection of birnavirus infection, in particular for the detection of IBDV infection in an animal comprising the step of examining a sample of the animal for the presence of VP5 antibodies or antigens. The animal is an animal from the field and is in particular an avian species, preferably a chicken. The sample coming from the animal may be any sample in which IBDV antibodies or antigens are present, e.g. a blood, serum or tissue sample, the serum sample being preferred.

A preferred method for determining birnavirus infection in an animal is a method for the detection of antibodies against the VP5 protein, comprising the steps of:
(i) incubating a sample suspected of containing anti-birnavirus antibodies, with VP5 antigen,
(ii) allowing the formation of antibody-antigen complex , and
(ii) detecting the presence of the antibody-antigen complex.

The design of this immunoassay may vary. For example, the immunoassay may be based upon competition or direct reaction. Furthermore, protocols may use solid supports or may use cellular material. The detection of the antibody-antigen complex may involve the use of labelled antibodies; the labels may be, for example, enzymes, fluorescent-, chemiluminescent-, radio-active- or dye molecules.

Suitable methods for the detection of the VP5 antibodies in the sample include the enzyme-linked immunosorbent assay (ELISA), immunofluorescent test (IFT) and Western blot analysis.

In an exemplifying ELISA, the wells of a polystyrene micro-titration plate are coated with VP5 antigen. Next, the wells of the coated plates are filled with chicken serum and serial dilutions are made. After incubation, chicken anti-VP5 protein serum antibodies are determined by detecting antibody (monoclonal or polyclonal) with the same specificity as the coated one, but which is labelled (e.g. with biotin). The labelled antibody will occupy the free antigens that have not been occupied by anti-VP5 antibodies in the chicken serum. For example, horse radish peroxidase coupled to avidin may be added and the amount of peroxidase is measured by an enzymatic reaction. If no antibodies against VP5 are present in the chicken serum sample then a maximum absorption is obtained. If the serum contains many antibodies against VP5 then a low absorption is expected. Alternatively, after the incubation with chicken serum, the amount of antibodies present in the serum that bound to the VP5 antigen may be determined directly by using an anti-chicken conjugate followed by the enzymatic reaction.

In a sandwich ELISA the wells of a polystyrene micro-titration plate can be coated with a monoclonal antibody directed against the VP5 protein. Next, the wells of these coated plates are incubated with VP5 antigen. After the antigen is captured, the wells are filled with the chicken serum and serial dilutions are made. Subsequently, the protocol as described above may be followed. This test can also be carried out by using polyclonal serum against VP5 instead of the coated monoclonal antibodies.

In another diagnostic test (Western blot analysis), the VP5 antigen (containing) material is subjected to SDS-PAGE. Next, the separated proteins are electroblotted onto nitro-cellulose membrane. Thereafter, the membranes can be cut into lanes and the lanes are incubated with the chicken serum. The presence of VP5 antibodies in the sample can be determined by examination whether antibodies bound to the VP5 antigen, for example by using an anti-chicken conjugate followed by an enzymatic reaction. If antibodies against VP5 are present then a band at about 17 kDa is identifiable.

The VP5 antigen may be any VP5 protein (fragment) comprising material which allows the formation of the VP5 antigen-VP5 antibody complex. Preferably, the VP5 antigen comprises the expression product of a conventional recombinant host cell or virus, e.g. such as E.coli expressed VP5 (Mundt et al., J. Gen. Virol. 76, 437-443, 1995) or baculovirus expressed protein (Vakharia et al., Vaccine 12, 452-456, 1994; Vakharia et al., J. Gen Virol. 74, 1201-1206, 1993). In a further embodiment of the present invention a diagnostic test kit is provided which is suitable for performing the diagnostic test according to the invention as described above.

In particular, a diagnostic test kit is provided which comprises in addition to the components usually present, the VP5 antigen (if desired coated onto a solid phase) as the immunological reagent. Other components usually present in such a test kit include, biotin or horseradish peroxidase conjugated antibodies, enzyme substrate, washing buffer etc.

To determine birnavirus VP5 antigen in a test sample from an animal in the field, VP5-specific antibodies are used as the immunological reagent, preferably fixed to a solid phase. The test sample is added, and after an incubation time allowing formation of the antibody-antigen complex, a second labelled antibody may be added to detect the complex.

### EXAMPLES

### Example 1.

### Construction and analysis of recombinant VP5⁻ IBD virus

### Construction of full length VP5⁻ clone of IBDV segment A.

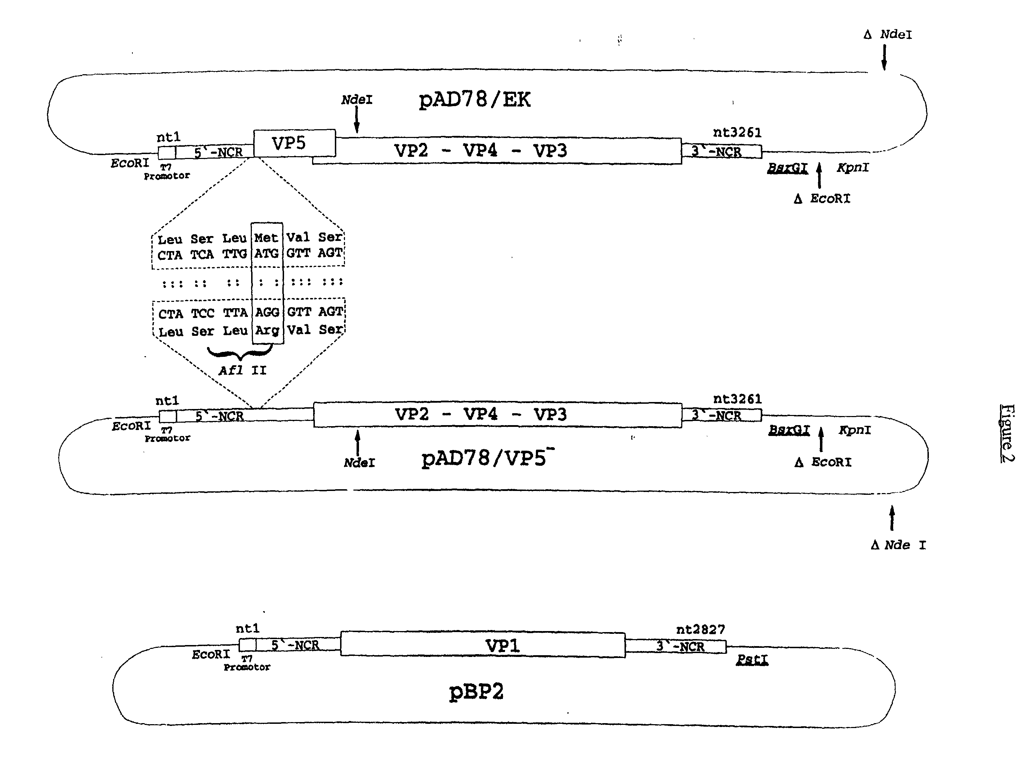

To construct a VP5-negative IBDV, the *Eco*RI site immediately following the 3'-end of the full length cDNA of strain D78 segment A (pUC19FLAD78; Mundt and Vakharia, Proc. Natl. Acad. Sci. USA 93, 11131-11136, 1996) was deleted. An *Eco*RI - *Kpn*I fragment containing the T7 polymerase binding site followed by the complete segment A sequence was excised and inserted into *Eco*RI - *Kpn*I cleaved vector pUC18 after inactivation of the unique *Nde*I within the vector sequence resulting in plasmid pAD78/EK. Thereafter, the genomic region encompassing the initiation codon for VP5 was amplified in two pieces using primers A1F5' and VP5MutR, and VP5MutF and A2R, respectively (see Table 1 for sequence and location of primers). PCR fragments were cloned separately and were subsequently fused via a unique *Afl*II site which had been created by mutations within respective primers (see Fig. 2). An *Eco*RI - *Nde*I fragment containing the T7 polymerase binding site, and the 5'-part of segment A including the introduced mutations was excised and used to substitute the wild-type *Eco*RI - *Nde*I fragment in pAD78/EK to yield plasmid pAD7δ/VP5⁻. Of the three mutations introduced one altered the initiation methionine codon for VP5 into an arginine codon (Fig. 2).

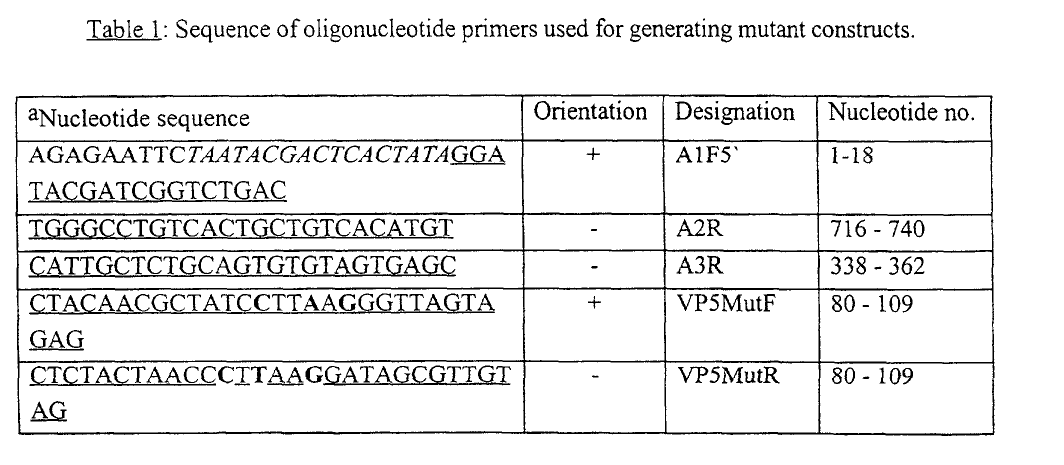

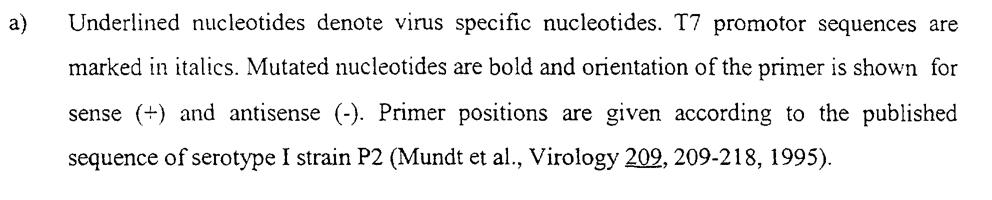

**Virus recovery from cRNA.** For *in vitro* transcription of RNA plasmids pAD78/EK, pAD78/VP5⁻ and pBP2 (Fig. 2) were linearized by cleavage with *Bsr*GI and *Pst*I, respectively. Treatment of linearized DNA, transcription and purification of RNA, and transfection were carried out as described by Mundt and Vakharia (1996, supra) with the exception that secondary CEC were used for the transfection experiments. Three days after transfection a CPE was visible in CEC. Cells were freeze/thawed, centrifuged at 700 x g to eliminate cellular debris, and the resulting supernatants were filtrated through 0.45 µm filters and stored at -20°C. For the transfection experiments full length cDNA clones of segment A of strain D78 capable of expressing (pAD78/EK) or unable to express VP5 (pAD78/VP5⁻) were transcribed into synthetic RNA and cotransfected with segment B full length cRNA into CEC. Resulting virus progeny IBDV/EK and IBDV/VP5⁻ was further characterised.

**Analysis of transfection progeny by immunofluorescence and Radioimmunoprecipitation assay (RIPA).** VP5 was expressed in E.coli as described in Mundt et al. (J. Gen. Virol. 76, 437-443, 1995). Rabbit monospecific polyclonal anti serum and mouse monoclonal antibodies against VP5 were prepared according to standard protocols. Vero cells infected with IBDV/VP5⁻, IBDV/EK, and non-infected cells , respectively, were incubated with rabbit anti-IBDV serum, rabbit anti-VP5 serum and with anti-VP5 mAb DIE 7, and stained with fluoresceine-conjugated secondary antibodies. Both antisera and the monoclonal antibody recognised IBDV antigens in the cytoplasm of IBDV/EK infected cells. In contrast, whereas the anti-IBDV serum readily detected viral antigens in IBDV/VP5⁻ infected cells, neither the monospecific anti VP5-serum nor the monoclonal anti-VP5 antibody exhibited specific reactivity. None of these immunological reagents reacted with non-infected controls.

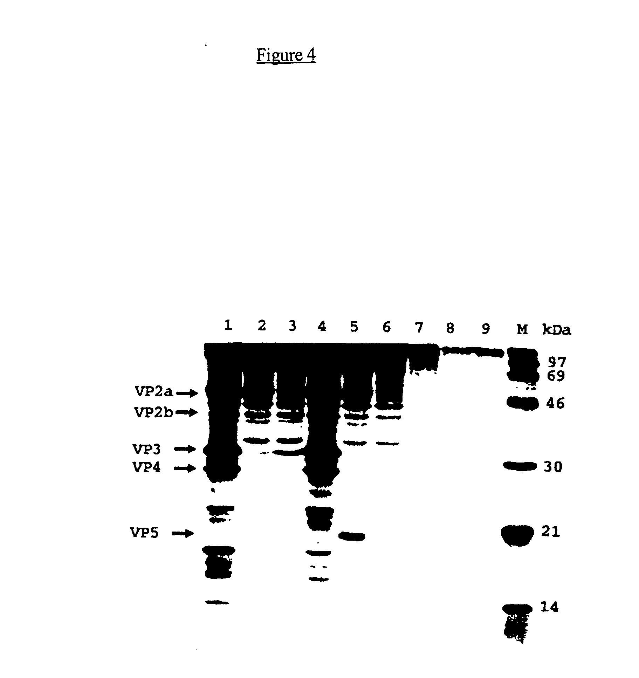

To analyse viral proteins expressed during replication lysates of radioactively labelled CEC infected with IBDV/VP5⁻ (Fig 4, lanes 1-3) and IBDV/EK (Fig. 4, lanes 4-6) were immunoprecipitated with rabbit anti-IBDV serum, rabbit anti-VP5 serum and mAb DIE 7. Non-infected CEC were used as control (Fig. 4, lanes 7-9). IBDV/EK (lane 4) as well as IBDV/VP5⁻ (lane 1) infected CEC showed viral proteins VP2, VP3, and VP4 after precipitation with rabbit anti-IBDV serum. The rabbit anti-VP5 serum (lane 5) and mAb DIE 7 (lane 6) precipitated VP5 with a molecular mass of 21 kDa only from IBDV/EK infected cells. No specific reactivity was detectable in IBDV/VP5⁻ infected CEC after precipitation with rabbit-anti VP5 (lane 2) as well as the VP5 specific mAb DIE 7 (lane 3). Non-infected CEC showed no specific reactivity (lanes 7-9).

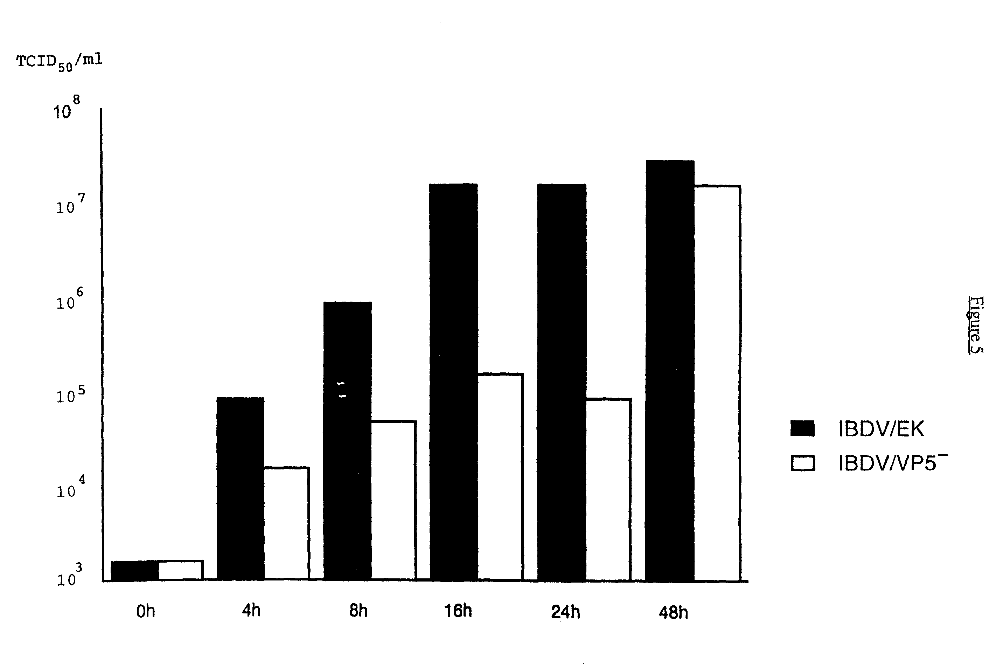

**Replication of IBDV/VP5**^{**-**} **in CEC.** To assay replication of IBDV/VP5⁻ in more detail one step growth was analysed (Fig. 5). Confluent secondary CEC were infected with IBDV/EK and IBDV/VP5⁻ with 10⁷² TCID₅₀, respectively. Immediately after overlaying the infected cells with 5 ml growth medium, supernatant from one infected CEC tissue plate of each virus was removed and stored at -20°C (0 h p.i.). Remaining tissue culture plates were further incubated and 4h, 8h, 16h, 24h, and 48h p.i. supernatants were removed and stored at -20°C. Supernatants were centrifuged and titrated according to standard methods. The TCID₅₀ at the different time points after infection showed that the VP5 expressing virus (IBDV/EK) replicated faster than the virus mutant lacking VP5 (IBDV/VP5⁻). 16 h after infection IBDV/EK showed a 100-fold higher than IBDV/VP5⁻ (Fig. 5). However, at 48 h p.i. IBDV/VP5⁻ reached a titre of 10^{7.2} TCID₅₀/ml which was similar to IBDV/EK (10 ^{7.45}/ml)

**Preparation of recombinant IBDV VP5**^{**-**}**-2.** Plasmid pAD78/VP5⁻-2 was prepared by techniques similar to those described above. The nucleotide sequence of part of the mutated VP5 gene is shown in SEQ ID No. 7 and Figure 3. A restriction enzyme fragment harbouring the mutations was used to substitute the wild-type *Eco*RI - *Nde*I fragment in pAD78/EK. An outline of the protocol for the preparation of the recombinant plasmid is shown in Figure 3. The organisation of pBD78 is also depicted in Figure 3. The recombinant virus was prepared as described above, except for the fact that segment B of strain D78 (SEQ ID No. 8) was used and QM-7 cells were used for the transfection experiment.

### Example 2

### Identification of VP5 protein in different IBDV strains

Different strains of IBDV were investigated for the expression of the VP5-gene. This was done by making use of the immuno-fluorescence technique (IFT). Chicken embryo fibroblasts grown in microtiterplates were infected with different IBDV strains. Three to 5 days after incubation at 37°C cells were fixed with 70% ethanol, then treated with polyclonal rabbit anti IBDV serum (R1928), polyclonal rabbit anti VP5 serum (RαVP5) or monoclonal antibody directed against VP5 (DIE7), respectively. Binding of the poly- or monoclonal antibodies to the different IBDV strains was visualised by making use of a fluorescence labelled conjugate (goat-anti-rabbit or goat-anti-mouse). The results are shown in Table 2:

**Table 2**

| Identification of different sero- and subtypes of IBDV strains. Determination of the presence of VP5 proteins. | | | | | |
|---|---|---|---|---|---|
| IBDV-serotype | IBDV-subtype | IBDV-strain | R1928 | RαVP5 | DIE7 |
| I | Classical | D78 | + | + | + |
| I | Classical | 228TC | + | + | + |
| I | Classical | PBG98 | + | + | + |
| I | Classical | Ram0404 | + | + | + |
| I | Classical | IBDV/EK | + | + | + |
| I | Classical | IBDV/VP5⁻ | + | - | - |
| I | GLS | GLS | + | + | + |
| I | Variant-E | 8903 | + | + | + |
| II | TY89 | TY89 | + | + | + |

From these data it can be concluded that the different strains of IBDV belonging to different sero- and subtypes do express the VP5-gene. Furthermore, the recombinant VP5⁻ IBDV vaccine strain can be differentiated from field and vaccine viruses, thereby enabling the recombinant VP5⁻ virus to be used as a marker vaccine.

### Example 3

### In vivo testing of the recombinant VP5⁺ and VP5⁻ IBDV vaccines in comparison with a commercial available live IBDV vaccine.

**Preparation of IBDV vaccine.** Primary chicken embryo fibroblast (CEF) cells were prepared at a final concentration of 2x10⁶/ml. The cells were cultured in Eagles minimum essential medium containing 5% fetal calf serum. To 25 ml of this cell suspension 0.1 ml IBDV/EK or IBDV/VP5⁻ virus (having an infectious titre of about 3.0 log10 TCID₅₀/ml) was added. After incubation for 5 days in a high-humidity incubator at 37°C, the total suspension was used in the animal experiment without further purification. The infectious titre of the supernantant was 10^{7.1} TCID50/ml.

**Animal experiment.** In this study the potency of different vaccines (VP5 positive strain IBDV/EK and a VP5 negative strain IBDV/VP5⁻, and the commercial available IBDV vaccine Nobilis strain D78, Intervet International B.V., NL) was investigated. SPF chicks of 3 weeks old were treated as indicated in the treatment schedule.
Treatment Schedule:

| Days after vaccination | Groups | | | |
|---|---|---|---|---|
| | 1 | 2 | 3 | 4 |
| 00 | IBDV/EK | IBDV/VP5⁻ | D78 | - |
| 03 | x | x1 | x | x |
| 07 | x,bl | x1,bl | x,b | x,bl |
| 14 | x,bl | x,bl | x,bl | x,bl |
| 20 | x,bl | x,bl | x,bl | x,bl |
| 21 | ch | ch | ch | ch |
| 24 | x | x | x | x |
| 31 | + | + | + | + |
| VP5⁺ Bursal disease vaccination with VP5 positive vaccine clone, eye-drop route, dose 10⁴⁶ TCID₅₀/animal, 0.1 ml/animal. VP5⁻ Bursal disease vaccination with VP5 negative vaccine clone, eye-drop route, dose 10⁵⁹ TCID₅₀/animal, 0.1 ml/animal. D78 Bursal disease vaccination with IBDV VACCINE NOBILIS STRAIN D78, eye-drop route, one field dose. ch Challenge with Bursal disease virus, Farragher strain F52/70, eye-drop route, dose 10^{2.0} CID₅₀/animal, 0.1 ml/animal. bl Serological examination; VN-test and/or Western blotting. x Histological examination (H.E. staining) and MCA-8 ELISA on bursae. x1 Histological examination (H.E. staining) and MCA-8 ELISA on bursae and reisolation of virus from bursa of Fabricius. + Clinical examination and after 10 days histological examination of the bursa. | | | | |

### Detection of virus in the bursa of Fabricius.

Three, 7, 14 and 20 days after eye-drop vaccination, animals were sacrificed and blood and bursae obtained. The presence of virus in the bursa was determined with an enzyme-linked immunosorbent assay (ELISA) making use of the monoclonal antibody 8 (MAB-8). MAB-8 is directed specifically against IBDV. Data are depicted in Table 3.

Furthermore, 3 and 7 days after vaccination, bursae from animals of group 2 were investigated for the presence of the recombinant VP5⁻ virus. For that purpose bursae were homogenised and cultured on chicken embryo fibroblasts. The presence of the VP5⁻ virus was determined by IFT using polyclonal rabbit sera against IBDV or VP5 or monoclonal antibodies against VP5. From 13 out of 15 bursae (87%) investigated, VP5⁻ virus could be reisolated and identified (positive for R1928 and negative for RαVP5 and DIE7). This indicates that the virus upon animal passage is still VP5⁻, indicating that the virus is stable and does not revert to VP5⁺. Furthermore, by using the different poly- and monoclonal antibodies VP5⁻ vaccine virus can be discriminated from all other vaccine and/or field IBDV viruses. Therefore, the VP5⁻ vaccine may be used as a marker vaccine.

Three days after challenge no virus could be detected in groups 1, 2 and 3 with the MCA-8 ELISA. In contrast, all animals of group 4 (non-vaccinated control group) contained challenge virus in the bursa of Fabricius, 3 days after challenge. The results show that animals vaccinated with recombinant VP5⁺ (group 1), recombinant VP5⁻ (group 2) and IBDV vaccine Nobilis D78 (group 3) were protected against severe challenge.

**Table 3**

| Individual data for detection of virus in the bursa of Fabricius with the MCA-8 ELISA at different days after vaccination or challenge. | | | | | | |
|---|---|---|---|---|---|---|
| | Days after vaccination→ | | | | Days after challenge | |
| | 3 | 7 | 14 | 20 | 3 | |
| Group↓ | Virus detection by ELISA↓ | | | | | Protection↓ |
| 1 VP5⁺ | 2/8* | 1/7 | 0/2 | 0/3 | 0/5 | 100% |
| 2 VP5⁻ | 0/8 | 0/7 | 0/2 | 0/3 | 0/5 | 100% |
| 3 D78 | 1/8 | 6/7 | 0/2 | 0/3 | 0/5 | 100% |
| 4 - | 0/8 | 0/7 | 0/2 | 0/3 | 5/5 | 0% |

| | | | | | | |
|---|---|---|---|---|---|---|
| *Number of positive bursae per total number tested. | | | | | | |

### Detection of lesions in the bursa of Fabricius.

The microscopic average lesion score induced by the different IBDV (recombinant) vaccines or the challenge virus are depicted in Table 4.

Before challenge, animals vaccinated with the recombinant VP5⁺ IBDV vaccine (group 1) or vaccinated with IBDV vaccine Nobilis D78 (group 3) showed mild to moderate lesions in the bursa. Three days after challenge only chronic lesions were observed in the bursa of Fabricius, indicating that the animals of groups 1 and 3 were protected against challenge. Furthermore, 10 days after challenge only very mild lesions (0-20% lymphocytic depletion) were observed in the bursa of the animals vaccinated with VP5⁺ recombinant IBDV vaccine or with Nobilis vaccine D78. In contrast animals not vaccinated and challenged showed severe lesions 10 days after challenge. In other words all animals (100%) of groups 1 and 3, vaccinated with the VP5⁺ recombinant IBDV vaccine or with Nobilis vaccine D78 were protected against severe challenge.

Three, 7, 14 and 20 days after vaccination and 3 and 10 days after challenge with the recombinant VP5⁻ IBDV vaccine, animals of group 2 showed no to hardly any lesions (0-20% lymphocytic depletion) in the bursa. All animals of group 2, vaccinated with the VP5⁻ recombinant IBDV vaccine, were protected against severe challenge. When animals vaccinated with the recombinant VP5⁻ IBDV vaccine are compared to animals of groups 1 or 3 (vaccinated with a recombinant VP5⁺ or commercial available vaccine) the recombinant VP5⁻ vaccine induces less lesions and therefore, is safer, milder than the vaccines tested in this experiment.

Three days post-challenge, all non-vaccinated animals of group 4 showed severe acute lesions in the bursa (total lymphocyte depletion, score 5.0). Ten days after challenge, all animals ( 17 out of 17 animals) showed total lymphocytic depletion, indicating that these animals were not protected against severe challenge. Animals that died after challenge, all showed severe lesions in the bursa of Fabricius. It was concluded that control group 4 was not protected against severe challenge indicating that the test conditions were optimal.

**Table 4**

| Average bursal lesion score at different days after vaccination or challenge. The average lesion score is calculated as follows: all lesion scores from the animals per group on a certain day are added. This number is then divided by the total number of animals investigated in that group on that day. Individual scores range from 1 to 5. Score 0 = no lymphocytic depletion, score 1 = 0 - 20%; score 2 = 20 - 40%; score 3 = 40 - 60%; score 4 = 60 - 80% and score 5 = 80 - 100 % lymphocytic depletion (total lymphocytic depletion). | | | | | | | |
|---|---|---|---|---|---|---|---|
| | Days after vaccination→ | | | | Days after challenge→ | | |
| | 3 | 7 | 14 | 20 | 3 | 10 | |
| Group↓ | Bursal lesions score↓ | | | | | | Protection↓ |
| 1 VP5⁺ | 0.8 | 2.9 | 1.0 | 1.0 | 1.0^{c} | 0.6 | 100% |
| 2 VP5⁻ | 0.0 | 0.0 | 0.5 | 0.0 | 0.0^{c} | 0.1 | 100% |
| 3 D78 | 0.1 | 2.4 | 3.5 | 2.0 | 2.8^{c} | 1.1 | 100% |
| 4 - | 0.0 | 0.0 | 0.0 | 0.0 | 5.0^{a} | 5.0 | 0% |

| | | | | | | | |
|---|---|---|---|---|---|---|---|
| ^{a} Acute lesions | | | | | | | |
| ^{c} Chronic lesions | | | | | | | |

### Serological response.

The serological response of the animals was determined by measuring the ability of blood serum to neutralise a classical infectious bursal disease virus strain in a virus neutralising (VN) test. Serum was investigated 3, 7, 14 and 20 days after vaccination. The average neutralising titres are shown in Table 5.

The results show that recombinant IBDV vaccine VP5⁺ applied to chickens of group 1 induced a good and high serological response 20 days after vaccination which is comparable to the serological response of the chickens vaccinated with the commercial IBDV vaccine Nobilis strain D78 (group 3). The recombinant IBDV vaccine VP5⁻ applied to chickens of group 2 induced also a good serological response. A titre of 9.4 log2 was observed 20 days after vaccination. The serological response induced by the recombinant VP5⁻ IBDV vaccine was delayed when compared to the serological response induced by the recombinant IBDV VP5⁺ vaccine or the commercial IBDV vaccine Nobilis strain D78.

The non-vaccinated group 4 showed no serological response to IBDV.

**Table 5**

| Average IBDV-VN-titres for groups 1 to 4 at different days after vaccination, expressed as log2 of the dilution. | | | | |
|---|---|---|---|---|
| Group | Days after vaccination | | | |
| | 3 | 7 | 14 | 20 |
| 1 VP5⁺ | ≤ 1.0 ± 0.0 | 7.1 ± 1.7 | 10.2 ± 1.4 | 11.9 ± 1.8 |
| 2 VP5⁻ | ≤ 1.0 ± 0.0 | 2.1 ± 1.7 | 6.3 ± 2.9 | 9.4 ± 1.4 |
| 3 D78 | ≤ 1.0 ± 0.0 | 5.2 ± 2.8 | 10.3 ± 1.3 | 11.6 ± 1.5 |
| 4- | ≤ 1.0 ± 0.0 | ≤ 1.0 ± 0.0 | ≤ 1.0 ± 0.0 | ≤ 1.0 ± 0.0 |

### Serological differentiation between antisera.

The serological response against VP5 was investigated by making use of western blot analysis. For this purpose the VP5 protein was expressed in the E. coli or baculo expression system. The expressed proteins were separated by SDS PAGE. Next the proteins were electroblotted onto a nitro-cellulose membrane. Thereafter, the membrane was cut into lanes and the lanes were incubated with rabbit anti-VP5 serum, chicken serum directed against VP5⁺ recombinant vaccine, chicken serum directed against VP5⁻ recombinant vaccine or negative serum from SPF chickens. Data are summarised in Table 6. As can be seen from Table 6, the VP5⁻ serum does not induce a serological response against VP5. In contrast the rabbit anti-VP5 serum and chicken serum directed against VP5⁺ recombinant vaccine do recognise the VP5-protein and thus induces a serological response against VP5. This indicates that chicken serum may be used to investigate if animals are exposed to a virus that expresses the VP5 protein (e.g. field virus) or to the VP5⁻ recombinant vaccine.

**Table 6**

| Western blot analysis. Serum from animals vaccinated with VP5⁺ or VP5⁻recombinant vaccine as well as SPF chicken serum and anti VP5-rabbit serum were investigated for their reaction with the VP5-protein. | |
|---|---|
| Identification of serum sample | Immuno-blot |
| VP5⁺ vaccinated animal, serum sample 20d after vaccination | positive |
| VP5⁻ vaccinated animal, serum sample 20d after vaccination | negative |
| Non-vaccinated control, serum sample at 20d | negative |
| Rabbit anti VP5 serum | positive |

### Mortality and clinical signs.

None of the animals vaccinated with VP5⁺ IBDV vaccine (group 1), vaccinated with recombinant VP5⁻ IBDV vaccine (group 2) or vaccinated with the commercial IBDV vaccine Nobilis strain D78 (group 3), died or showed clinical signs of infectious bursal disease after challenge, indicating that the animals were protected against severe challenge. All animals in the non-vaccinated control group were not protected against severe challenge.

### Example 4

### In vivo testing of the recombinant VP5⁻-2 vaccine

**Preparation of the IBDV vaccines.** Primary chicken embryo fibroblasts (CEF) cells were prepared at a final concentration of 2 x 10⁶/ml. The cells were cultured in Eagles minimum essential medium containing 5% fetal calf serum. To 15 ml of this cell suspension 0.1 ml IBDV/VP5⁻-2 (D78/D78/VP5⁻) virus was added. After incubation for 6 days in a high humidity incubator at 37°C, the supernatant was titrated. The infectious titre of the supernatant was 10^{8.2} TCID₅₀/ml. For the second animal experiment the supernatant was diluted to result in a vaccine dose of 10^{5.5} TCID₅₀/animal and for the first animal experiment the supernatant was diluted to result in a vaccine dose of 10^{4.0} TCID₅₀/animal or 10^{5.0} TCID₅₀/egg.

**First animal experiment.** The effect of the vaccine is assessed by measurement of the serological response and resistance to challenge obtained from administering a challenge virus at the age of 14 days. The vaccine (10^{5.0} TCID₅₀/egg or 10^{4.0} TCID₅₀/animal of D78/D78/VP5⁻) was applied *in ovo* or intramuscularly at day old. Microscopic lesions in the bursa were investigated, 3 and 10 days after challenge. Protection against challenge was determined and the serological response at the age of 14 days old was determined with the VN-test.

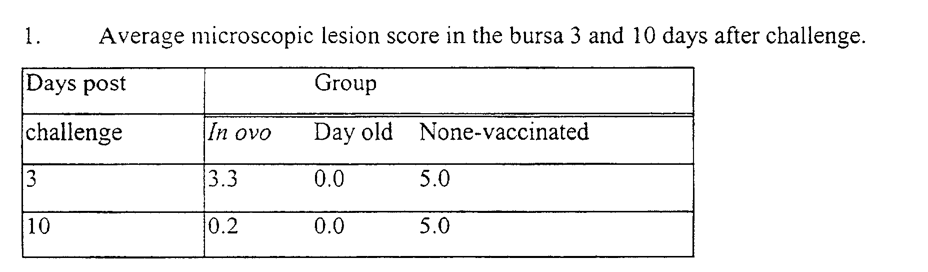

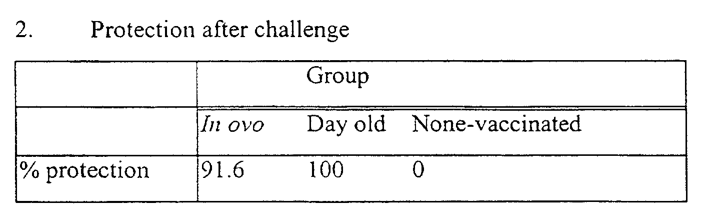

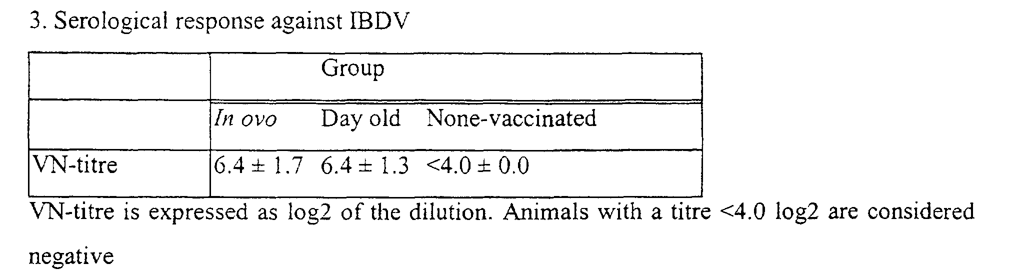

### Conclusions

- 1: The D78/D78/VP5⁻ strain is a highly attenuated IBD-virus
- 2: The virus strain is very mild
- 3: The virus can induce a serological response
- 4: The virus can induce protection
- 5: The virus strain can be applied by intramuscular injection to 1 day old SPF chickens and *in ovo* to 18-days-old embryonated SPF-eggs

**Second animal experiment.** The effect of the vaccine is assessed by measurement of the serological response against IBDV and resistance to challenge obtained from administering a challenge virus, 21 days after administering the Gumboro vaccine. The vaccine (10^{5.5} TCID₅₀/animal of D78/D78/VP5⁻) was applied via the intramuscular route to 14 days old SPF-chickens. Three, 7, 14, and 20 days after vaccination and 3 days after challenge Bursa, spleen, thymus, liver, duodenum, pancreas, ceacal tonsils and harderian gland were investigated for microscopic lesions. Ten days after challenge Bursae were investigated for microscopic lesions. Sera were tested in the VN-test. And mortality was scored after challenge.

| 1. Percentage mortality after challenge: | |
|---|---|
| | Mortality after challenge |
| Vaccinated group | 0% |
| Control group | 50% |

| 2. Microscopic lesions of the vaccinated group before and after challenge: | | | | | | | | |
|---|---|---|---|---|---|---|---|---|
| Days post | Bursa | Spleen | Thymus | Liver | Duodeum | Pancreas | Ceacal | Harderian |
| Vaccinat. | | | | | | | Tonsils | Gland |
| 3 | 0 | 0 | 0 | 0 | 0 | 0 | 0 | 0 |
| 7 | 0 | 0 | 0 | 0 | 0 | 0 | 0 | 0 |
| 14 | 0 | 0 | 0 | 0 | 0 | 0 | 0 | 0 |
| 20 | 0 | 0 | 0 | 0 | 0 | 0 | 0 | 0 |
| 24 | 0,A | 0 | 0 | 0 | 0 | 0 | 0 | 0 |
| 31 | 0,A | ND | ND | ND | ND | ND | ND | ND |
| A = None vaccinated animals showed a lymphocytic depletion score of 5.0 (100%) and 4.25, 3 and 10 days after challenge, respectively. ND = not done. | | | | | | | | |

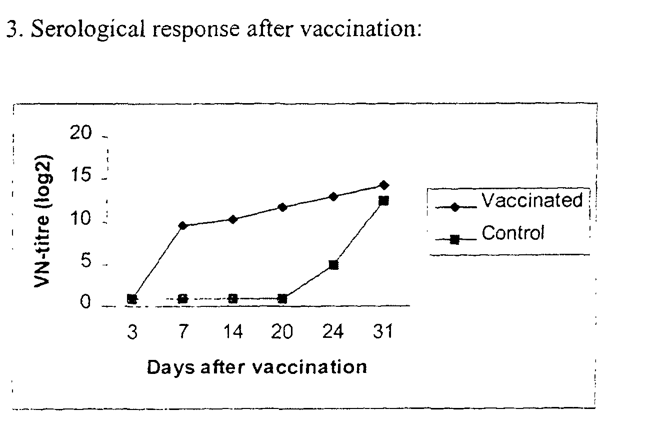

### Conclusions

- 1.: The D78/D78/VP5⁻ strain is a highly attenuated IBD-virus
- 2.: The virus strain is very mild and does not induce lesions in organs
- 3.: The virus can induce a serological response
- 4.: The virus can induce protection

### LEGENDS TO THE FIGURES

Figure 1 Genomic organization of segment A and segment B of IBDV. The numbers indicate the nucleotide positions of the start, end and coding region on the segments.

Figure 2 Construction of genomic cDNA clones for the preparation of IBDV/VP5⁻. Plasmid pAD78/EK contains the complete D78 segment A cDNA encoding the polyprotein (VP2-VP4-VP3) and VP5. Plasmid pBP2 contains the complete strain P2 segment B encoding VP1. Mutations were introduced in plasmid pAD78/VP5⁻ altering the methionine start codon for VP5 into arginine and creating an artificial Afl II cleavage site. Recombinant plasmids were linearized with the underlined restriction enzymes, followed by T7 polymerase transcription.

Figure 3 Construction of genomic cDNA clones for the preparation of IBDV/VP5⁻-2. Plasmid pAD78/EK contains the complete D78 segment A cDNA encoding the polyprotein (VP2-VP4-VP3) and VP5. Plasmid pBD78 contains the complete strain D78 segment B encoding VP1. Mutations were introduced in plasmid pAD78/VP5⁻ altering the methionine start codon for VP5 into glutamic acid and creating an artificial BstBI cleavage site. Further mutations were introduced in the arginine and glutamine codon. Recombinant plasmids were linearized with the underlined restriction enzymes, followed by T7 polymerase transcription.

Figure 4 Radioimmunoprecipitation of proteins from CEC infected cells with recombinant IBDV. CEC infected cells with IBDV/VP5⁻ (lanes 1-3), IBDV/EK (lanes 4-6) and uninfected controls were immunoprecipitated with rabbit anti-IBDV serum (lanes 1, 4, 7), rabbit anti-VP5 serum (lanes 2, 5, 8) and mAb DIE 7 (lanes 3, 6, 9). Position of molecular mass markers (M) is indicated. Location of the viral proteins VP2, VP3, VP4 and VP5 are marked.

Figure 5 Replication kinetics of IBDV/EK and IBDV/VP5⁻. Infectious titers of supernatants (vertical axis) are determined at the times indicated.

## Claims

1. A birnavirus mutant which is not able to produce a native VP5 protein as a result of a mutation in the VP5 gene of the birnavirus genome, **characterised in that** the mutation comprises:
(i) a substitution of at least two nucleotides of the start codon of the VP5 gene, and
(ii) a stop codon in each of the three open reading frames in the 5'-end of the VP5 gene.

2. A birnavirus mutant according to claim 2, **characterised in that** the birnavirus is infectious bursal disease virus (IBDV).

3. A birnavirus mutant according to claims 1-2, **characterised in that** the mutation is in the genome of a virulent field virus.

4. A birnavirus mutant according to claim 2, **characterised in that** the mutation is in the genome of a vaccine strain, preferably in vaccine strain D78.

5. A birnavirus mutant according to claims 2-4, **characterised in that** the mutant has a mutated start codon and three stop codons in the 5'-end of the VP5 gene as shown in SEQ ID No: 7.

6. A birnavirus mutant according to claims 2-5, **characterised in that** the IBDV expresses a chimeric VP2 protein comprising virus neutralising epitopes of different antigenic IBDV types.

7. A vaccine against a birnavirus infection in animals, **characterised in that** it comprises a birnavirus mutant according to claims 1-6 and a pharmaceutically acceptable carrier.

8. A method for the attenuation of virulence of a birnavirus in an animal, comprising the step of introducing a mutation in the VP5 gene as a result of which the birnavirus is not able to produce a VP5 protein.

9. A method according to claim 9, wherein the mutation is a substitution.

10. A method according to claims 8-9, wherein the birnavirus is infectious bursal disease virus (IBDV).

11. A method according to claims 8-10, wherein the mutation is in the genome of a virulent field virus.

12. A method according to claims 8-11, wherein the mutation comprises a substitution of at least two nucleotides of the start codon of the VP5 gene.

13. A method according to claim 12 wherein the mutation comprises additionally one or more stop codons in the 5'-end of the VP5 gene.

14. A method according to claim 13, wherein the mutation comprises a stop codon in each of the three open reading frames.

15. A method according to claim 14, wherein the mutation is in the start codon and comprises three stop codons in the 5'-end of the VP5 gene as shown in SEQ ID No: 7.

## Patentansprüche

1. Eine Birnavirus Mutante, die aufgrund einer Mutation im VP5 Gen des Birnavirus Genoms kein natives VP5 Protein produzieren kann, **dadurch gekennzeichnet, dass** die Mutation:
(i) eine Substitution von mindestens zwei Nukleotiden des Startcodons des VP5 Gens, und
(ii) ein Stopcodon in jedem der drei offenen Leserahmen am 5'-Ende des VP5 Gens.
umfasst.

2. Eine Birnavirus Mutante gemäss Anspruch 1, **dadurch gekennzeichnet dass** das Birnavirus ein Infektiöses Bursitis Virus (IBDV) darstellt.

3. Eine Birnavirus Mutante gemäss Ansprüchen 1-2, **dadurch gekennzeichnet, dass** die Mutation im Genom eines virulenten Feldvirus ist.

4. Eine Birnavirus Mutante gemäss Anspruch 2, **dadurch gekennzeichnet, dass** die Mutation im Genom eines Impfstoff-Stammes, vorzugsweise im Impfstoff-Stamm D78, ist.

5. Eine Birnavirus Mutante gemäss Ansprüchen 2-4, **dadurch gekennzeichnet, dass** die Mutante ein mutiertes Startcodon und drei Stopcodons am 5'-Ende des VP5-Gens wie in SEQ ID No:7 dargestellt besitzt.

6. Eine Birnavirus Mutante gemäss Ansprüchen 2-5, **dadurch gekennzeichnet, dass** das IBDV ein chimäres VP2 Protein exprimiert, das Virus-neutralisierende Epitope von unterschiedlichen, antigenen IBDV-Typen umfasst.

7. Ein Impfstoff gegen eine Birnavirus Infektion in Tieren, **dadurch gekennzeichnet, dass** er eine Birnavirus Mutante gemäss Ansprüchen 1-6 und einen pharmazeutisch verträglichen Träger umfasst.

8. Verfahren zur Abschwächung der Virulenz eines Birnavirus in einem Tier, welches den Schritt des Einführens einer Mutation in das VP5 Gen umfasst, als Folge dessen das Birnavirus kein VP5 Protein produzieren kann.

9. Verfahren gemäss Anspruch 8, worin die Mutation eine Substitution darstellt.

10. Verfahren gemäss Ansprüchen 8-9, worin das Birnavirus ein Infektiöses Bursitis Virus (IBDV) darstellt.

11. Verfahren gemäss Ansprüchen 8-10, worin die Mutation im Genom eines virulenten Feldvirus ist.

12. Verfahren gemäss Ansprüchen 8-11, worin die Mutation eine Substitution von mindestens zwei Nukleotiden des Startcodons des VP5 Gens umfasst.

13. Verfahren gemäss Anspruch 12, worin die Mutation zusätzlich ein oder mehrere Stopcodons am 5'-Ende des VP5-Gens umfasst.

14. Verfahren gemäss Anspruch 13, worin die Mutation in jedem der drei offenen Leserahmen ein Stopcodon umfasst.

15. Verfahren gemäss Anspruch 14, worin die Mutation im Startcodon ist und drei Stopcodons am 5'-Ende des VP5-Gens wie in SEQ ID No:7 dargestellt umfasst.

## Revendications

1. Un mutant du birnavirus qui est incapable de produire une protéine VP5 native, résultant d'une mutation dans le gène VP5 du génome du birnavirus, **caractérisé en ce que** la mutation comprend :
i) une substitution d'au moins deux nucléotides du codon de départ du gène VP5, et
ii) un codon d'arrêt dans chacun des trois cadres ouverts de lecture dans l'extrémité 5' du gène VP5.

2. Un mutant du birnavirus selon la revendication 1, **caractérisé en ce que** le birnavirus est le virus de la bursite infectieuse aviaire (IBDV : *Infections Bursal Diseuse Virus*).

3. Un mutant du birnavirus selon la revendication 1 ou 2, **caractérisé en ce que** la mutation est dans le génome d'un virus sauvage virulent.

4. Un mutant du birnavirus selon la revendication 2, **caractérisé en ce que** la mutation est dans le génome d'une souche de vaccin, de préférence dans la souche de vaccin D78.

5. Un mutant du birnavirus selon l'une des revendications 2 à 4, **caractérisé en ce que** le mutant a un codon de départ ayant subi une mutation et les trois codons d'arrêt dans l'extrémité 5' du gène VP5 tel que représenté dans la SEQ ID n° 7.

6. Un mutant du birnavirus selon l'une des revendications 2 à 5, **caractérisé en ce que** l'IBDV exprime une protéine chimère VP2 comprenant des épitopes neutralisant des virus de différents types antigènes d'IBDV.

7. Un vaccin contre une infection par le birnavirus chez des animaux, **caractérisé en ce qu'**il comprend un mutant du birnavirus selon l'une des revendications 1 à 6 et un support pharmaceutiquement acceptable.

8. Un procédé pour atténuer la virulence d'un birnavirus chez un animal, comprenant l'étape consistant à introduire une mutation dans le gène VP5 pour que le birnavirus soit incapable de produire une protéine VP5.

9. Un procédé selon la revendication 9, dans lequel la mutation est une substitution.

10. Un procédé selon la revendication 8 ou 9, dans lequel le birnavirus est le virus de la bursite infectieuse aviaire (IBDV).

11. Un procédé selon l'une des revendications 8 à 10, dans lequel la mutation est dans le génome d'un virus sauvage virulent.

12. Un procédé selon l'une des revendications 8 à 11, dans lequel la mutation comprend une substitution d'au moins deux nucléotides du codon de départ du gène VP5.

13. Un procédé selon la revendication 12, dans lequel la mutation comprend en outre un ou plusieurs codons d'arrêt dans l'extrémité 5' du gène VP5.

14. Un procédé selon la revendication 13, dans lequel la mutation comprend un codon d'arrêt dans chacun des trois cadres ouverts de lecture.

15. Un procédé selon la revendication 14, dans lequel la mutation est située dans le codon de départ et comprend trois codons d'arrêt dans l'extrémité 5' du gène VP5 tel que représenté dans la SEQ ID n° 7.
